# EUROPEAN PATENT SPECIFICATION

(11) **EP 4 081 651 B1**
(45) Date of publication and mention of the grant of the patent: **25.02.2026**
(21) Application number: 20905228.1
(22) Date of filing: 26.12.2020
(51) Int. Cl.: A61K 31/18, C12Q 1/68, A61P 17/00

(54) **BELINOSTAT INCREASING THE EXPRESSION OF MIR-335 AND ITS USE IN TREATING ATOPIC DERMATITIS**
BELINOSTAT ERHÖHT DIE EXPRESSION VON MIR-335 UND SEINE VERWENDUNG BEI DER BEHANDLUNG DER ATOPISCHEN DERMATITIS
BELINOSTAT AUGMENTANT L'EXPRESSION DE MIR-335 ET SON UTILISATION DANS LE TRAITEMENT DE LA DERMATITE ATOPIQUE

(30) Priority: 26.12.2019 SG 10201913182P
(43) Date of publication of application: 02.11.2022
(73) Proprietor: Agency for Science, Technology and Research, Singapore 138632 (SG)
(72) Inventor: SAMPATH, Prabha, Singapore 8A Biomedical Grove, 06-06 Immunos Singapore 138648 (SG); QUAH, Shan, Singapore 8A Biomedical Grove, 06-06 Immunos Singapore 138648 (SG)
(74) Representative: Boult Wade Tennant LLP
(86) International application number: PCT/SG2020/050784
(87) International publication number: WO 2021/133260

(56) References cited:
- WO-A1-2017/058007
- WO-A2-2008/142567
- JP-A- 2014 172 904
- US-A1- 2017 233 814
- CHIY LIEW WEN: "A THESIS SUBMITED FOR THE DEGREE OF DOCTOR OF PHILOSOPHY (SoM)", 24 January 2018 (2018-01-24), XP093108962, Retrieved from the Internet <URL:https://scholarbank.nus.edu.sg/handle/10635/142723>
- SONKOLY ENIK� ET AL: "MicroRNAs: Novel Regulators Involved in the Pathogenesis of Psoriasis?", PLOS ONE, vol. 2, no. 7, 11 July 2007 (2007-07-11), US, pages e610, XP093108785, ISSN: 1932-6203, DOI: 10.1371/journal.pone.0000610
- WEBER MICHEL J: "New human and mouse microRNA genes found by homology search", THE FEBS JOURNAL, WILEY-BLACKWELL PUBLISHING LTD, GB, vol. 272, no. 1, 2 December 2004 (2004-12-02), pages 59 - 73, XP002555647, ISSN: 1742-464X, [retrieved on 20041202], DOI: 10.1111/J.1432-1033.2004.04389.X
- SONKOLY, E. ; JANSON, P. ; MAJURI, M.L. ; SAVINKO, T. ; FYHRQUIST, N. ; EIDSMO, L. ; XU, N. ; MEISGEN, F. ; WEI, T. ; BRADLEY, M. : "MiR-155 is overexpressed in patients with atopic dermatitis and modulates T- cell proliferative responses by targeting cytotoxic T lymphocyte-associated antigen 4", JOURNAL OF ALLERGY AND CLINICAL IMMUNOLOGY, vol. 126, no. 3, 1 September 2010 (2010-09-01), pages 581 - 589, XP027399148, ISSN: 0091-6749, DOI: 10.1016/J.JACI. 2010.05.04 5
- FANG WANG, LING LI, KLAUS PIONTEK, MASAZUMI SAKAGUCHI, FLORIN M. SELARU: "Exosome - miR-335 as a novel therapeutic strategy in hepatocellular carcinoma", HEPATOLOGY, vol. 67, no. 3, 1 March 2018 (2018-03-01), pages 940 - 954, XP055834920, ISSN: 0270-9139, DOI: 10.1002/hep.29586
- MICHAŁ ROŻALSKI, RUDNICKA LIDIA, SAMOCHOCKI ZBIGNIEW: "MiRNA in atopic dermatitis", ADV DERMATOL ALLERGOL, vol. 3, 1 June 2016 (2016-06-01), pages 157 - 162, XP055565819, ISSN: 1642-395X, DOI: 0.5114/ADA.2016.60606
- LIEW, W.C. ET AL.: "Belinostat resolves skin barrier defects in atopic dermatitis by targeting the dysregulated miR-335:SOX6 axis", JOURNAL OF ALLERGY AND CLINICAL IMMUNOLOGY, vol. 146, no. 3, 1 September 2020 (2020-09-01), pages 606 - 620, XP055834921, ISSN: 0091-6749, DOI: 10.1016/J.JACI.2020.02.007

## Description

### FIELD

This invention relates to the fields of medicine, cell biology, molecular biology and genetics. This invention also relates to the field of medicine.

### BACKGROUND

Atopic dermatitis (AD) is a chronic inflammatory skin condition [1, 2], which affects 15-30% of children and 2-10% of adults. Generally referred to as "eczema", AD is a highly prevalent chronic relapsing inflammatory skin condition or disease which seriously affects patients' quality of life.

Symptoms include repeated episodes and remissions of skin inflammation, itching, scaling, and infection susceptibility, which severely diminish patients' quality of life. Key features of lesional skin from AD patients include skin barrier defects, compromised cornified envelope formation, and aberrant keratinocyte differentiation [3, 4]. The morphology and distribution of these skin lesions, which may occur anywhere on the body, is dependent on age and disease severity [5]. In infants, AD lesions are typically seen on the face, scalp, and extensor surfaces. Children aged 2-12 years old often have polymorphous manifestations with different types of skin lesions in the flexural areas, neck, dorsum of feet and hands [6]. In adolescents and adults, lesions are generally lichenified and present on flexural surfaces of extremities [7].

Whilst skin lesions represent the most noticeable symptom of atopic dermatitis, much lies beneath the surface. AD is a complex systemic disease which is often the first indicator of 'atopic march' - the progression from atopic dermatitis to asthma and allergic rhinitis in affected individuals. [8, 9]. In this model, it is postulated that the epidermal barrier defect in AD causes excessive exposure to environmental aeroallergens, and that this allergen sensitization initiates the atopic march [10]. The key role of epidermal barrier dysfunction in the origin of AD is a relatively recent finding; AD was initially thought to develop via immune dysregulation, with the skin barrier defect resulting from local inflammation [11]. It is now known that a strong genetic association exists between AD and filaggrin (FLG) loss-of-function mutations [12]. FLG is a protein involved in cornified envelope formation and barrier function. AD lesional skin also shows reduced expression of other barrier function proteins such as loricrin and involucrin [13]. The evidence that a skin barrier defect predisposes towards AD presents an interesting opportunity for new therapeutic approaches to AD and atopy as existing treatments are strictly symptomatic.

Chiy Liew Wen, 2018 (Chiy Liew Wen: "ROLE OF MICRORNAS IN THE PATHOGENESIS OF ATOPIC DERMATITIS" In: "A THESIS SUBMITED FOR THE DEGREE OF DOCTOR OF PHILOSOPHY (SoM)", 24 January 2018 (2018-01-24), DEPARTMENT OF BIOCHEMISTRY NATIONAL UNIVERSITY OF SINGAPORE, XP093108962, https://scholarbank.nus.edu.sg/handle/10635/142723) relates to the role of microRNAs in the pathogenesis of atopic dermatitis.

WO 2008/142567 A2 relates to microRNA molecules (miRNAs) which are associated with inflammatory skin disorders, such as psoriasis and atopic eczema.

### SUMMARY

The development and maintenance of a healthy skin barrier is dependent on a number of factors, including translational control by microRNAs.

MicroRNAs (miRNAs) are small, non-coding RNAs that mediate post-transcriptional gene regulation by targeting mRNAs for degradation and/or translational inhibition. The critical roles of miRNAs in mammalian skin development have been revealed by the defective skin phenotypes displayed by mice with a skin-specific knockout of *Dicer* and *Dgcr8,* which are two key components in the miRNA biogenesis pathway. Epidermal-specific deletion of *Dicer* and *Dgcr8* resulted in similar but abnormal skin phenotypes, such as reduced barrier function, defective hair follicle (HF) morphogenesis and keratinocyte hyperproliferation [14-16].

These findings present a strong case for miRNA involvement in skin differentiation, but stop short of identifying key miRNAs required for effective skin barrier function.

The invention provides an agent capable of up-regulating the expression or activity of miR-335 such as an miR-335 agonist, for use in a method of treatment, prophylaxis or alleviation of atopic dermatitis, wherein the agent comprises belinostat (PubChem CID: 6918638).

The agent may be formulated as a pharmaceutical composition comprising the agent together with a pharmaceutically acceptable excipient, carrier or diluent.

The agent may be formulated to be applied topically

The invention also provides belinostat (PubChem CID: 6918638) for use in the treatment, prophylaxis or alleviation of atopic dermatitis.

The invention also provides an *in vitro* or *ex vivo* method of up-regulating the expression of miR-335 in a cell, the method comprising exposing the cell to belinostat (PubChem CID: 6918638).

Described herein, but not part of the present invention, is miR-335 for use in a method of diagnosis, treatment, prophylaxis or alleviation of a dermatological condition such as atopic dermatitis.

miR-335 may comprise a polynucleotide sequence having miRBase Accession Number MI0000816. It may comprise a variant, homologue, derivative or fragment thereof. Such a variant, homologue, derivative or fragment thereof may comprise a sequence having 95%, 96%, 97%, 98% or 99% sequence identity to a polynucleotide sequence having miRBase Accession Number MI0000816. Such a variant, homologue, derivative or fragment may comprise miR-335 activity.

There is described herein an agent capable of up-regulating the expression or activity of miR-335 for use in a method of treatment, prophylaxis or alleviation of a dermatological condition such as atopic dermatitis. Such an agent may comprise an miR-335 agonist.

The agent may comprise a histone deacetylase (HDAC) inhibitor.

According to the present invention, the agent comprises belinostat (PubChem CID: 6918638) and is for use in a method of treatment, prophylaxis or alleviation of atopic dermatitis.

The invention provides a pharmaceutical composition comprising an agent as set out above together with a pharmaceutically acceptable excipient, carrier or diluent.

The pharmaceutical composition may be formulated to be applied topically.

Described herein, but not part of the present invention, is use of a histone deacetylase (HDAC) inhibitor such as belinostat (PubChem CID: 6918638) in the preparation of a medicament for the restoration of barrier function.

Described herein, but not part of the present invention, is a method of diagnosis of a dermatological condition such as atopic dermatitis (AD) in an individual. The method may comprise detecting the activity or expression level of miR-335 in a sample of or from an individual.

A decreased activity or expression level of miR-335 as compared to the activity or expression level of miR-335 in individual known not to be suffering from a dermatological condition such as atopic dermatitis may indicate that the individual is suffering, or is likely to be suffering, from a dermatological condition such as atopic dermatitis.

An expression level of 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, 5% or less of miR-335, as compared to the expression level of that miR-335 in or of an individual known not to be suffering from a dermatological condition such as atopic dermatitis, may be indicative of a dermatological condition such as atopic dermatitis.

The sample may comprise a skin sample.

There is provided, as defined herein, an agent for use in a method of treatment of atopic dermatitis in an individual. The method may comprise performing a method as set out above. Where the individual is determined to be suffering from, or likely to suffer from atopic dermatitis, the method may comprise administering to the individual a treatment for atopic dermatitis.

There is provided, as defined herein, an agent for use in a method for treating atopic dermatitis in an individual. The method may comprise obtaining the results of an analysis of the expression level of miR-335 or a variant, homologue, derivative or fragment thereof such as a sequence having at least 95%, 96%, 97%, 98% or 99% sequence identity thereto in a sample of or from an individual.

The method may further comprise administering a treatment for atopic dermatitis to the individual if the expression level of miR-335 is below a reference expression level, the reference expression level being the expression level of miR-335 in a sample of or from an individual known not to be suffering from a dermatological condition such as atopic dermatitis.

Described herein, but not part of the invention, is a kit for detecting a dermatological condition such as atopic dermatitis in an individual or susceptibility of the individual to a dermatological condition such as atopic dermatitis. The kit may comprise means for detection of the activity or expression level of miR-335 in the individual or a sample taken from him or her. The means for detection may comprise an miR-335 polynucleotide or a fragment thereof or a complementary nucleotide to a an miR-335 polynucleotide or a fragment thereof.

The practice of this invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies: A Laboratory Manual: Portable Protocol NO. I by Edward Harlow, David Lane, Ed Harlow (1999, Cold Spring Harbor Laboratory Press, ISBN 0-87969-544-7); Antibodies: A Laboratory Manual by Ed Harlow (Editor), David Lane (Editor) (1988, Cold Spring Harbor Laboratory Press, ISBN 0-87969-314-2), 1855. Handbook of Drug Screening, edited by Ramakrishna Seethala, Prabhavathi B. Fernandes (2001, New York, NY, Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3.

### BRIEF DESCRIPTION OF THE FIGURES

Figure 1A to Figure 1G are drawings showing a screen of miRNAs in AD identifies miR-335 as an epithelial differentiation factor.
Figure 1A is a drawing showing a scatter plot representing relative miR-335 levels in healthy unaffected skin samples (control, n=7) versus lesional skin from AD patients (AD-LS, n=10).

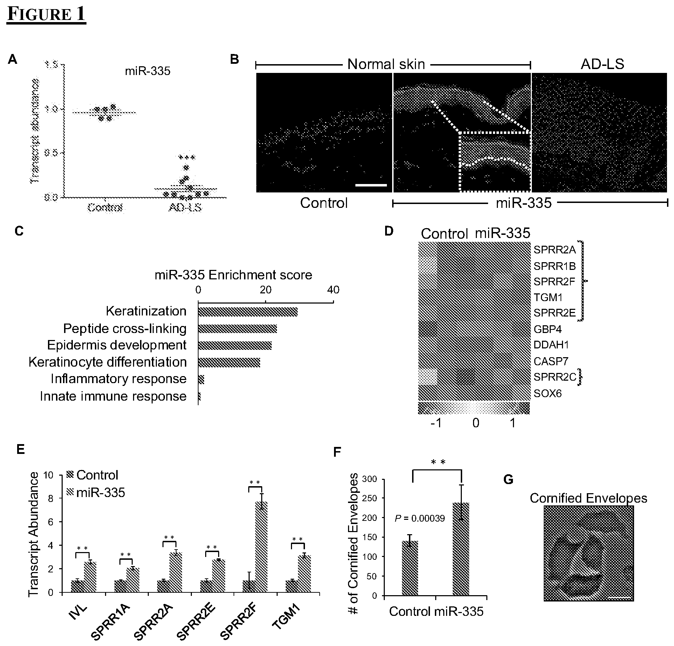

Figure 1B is a drawing showing expression of miR-335 detected by *in situ* hybridization in healthy unaffected versus AD lesional skin using specific probes against mature miR-335 or control probe. Inset in the middle panel of healthy skin displays the magnified view of epidermis showing supra-basal specific expression of miR-335. The middle panel of unaffected skin shows suprabasal expression of miR-335, with higher magnification inset. The basal layer is demarcated by dotted lines. The basal layer is demarcated by dotted lines. miR-335 is significantly downregulated in AD lesional skin (right panel). Scale bars, 100 µm.
Figure 1C is a drawing showing gene ontology analysis on differentially regulated genes in N/TERT-1 cells expressing miR-335 in comparison with control cells.
Figure 1D is a drawing showing a heat-map of selected genes generated from microarray using RNA from N/TERT-1 cells transfected with miR-335 mimics or control RNA. Expression values displayed in shades of red (high) or blue (low) relative to the individual mean value of the gene in linear scale.
Figure 1E is a drawing showing relative transcript abundance of IVL, TGM1 and SPRRs, all associated with keratinocyte differentiation, in N/TERT-1 cells. Cells transfected with miR-335 mimics or control RNA, represented as a bar graph.
Figure 1F is a drawing showing a bar graph representing the frequency of cornified envelopes per field in a cornified envelope assay of N/TERT-1 cells, on N/TERT-1 cells transfected with miR-335 mimics or control RNA.
Figure 1G is a drawing showing representative phase contrast microscopic image of mature cornified envelopes. Student's t-test was used to calculate *p* value and error bars denote ± s.e.m. **P<0.05 **P<0.01.* NS- non-significant.
Figure 2A to Figure 2D are drawings showing that SOX6 is a direct target of miR-335

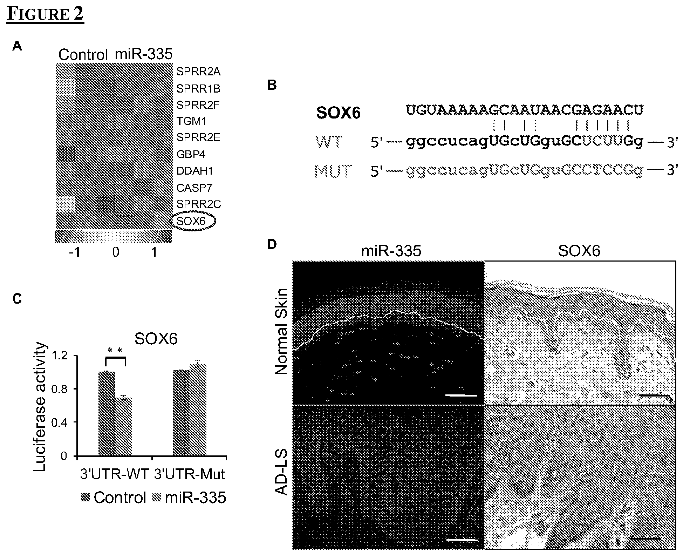

Figure 2A is a drawing showing a heat-map of selected genes generated from microarray using RNA from N/TERT-1 cells, transfected with miR-335 mimics or control RNA. Expression values displayed in shades of red (high) or blue (low) relative to the individual mean value of the gene in linear scale.
Figure 2B is a drawing showing sequences of miR-335 binding site in the 3' UTR of SOX6. For the generation of mutant 3' UTR lacking miR-335 binding site, the seed sequence was modified to sequence shown in red.
Figure 2C is a drawing showing validation of direct targets of miR-335 by luciferase reporter assays. Cells were co-transfected with wild-type (WT) or mutant (Mut) 3'-UTR luciferase reporter constructs as indicated, and either with miR-335 or non-targeting scrambled control. Normalized relative luciferase activities are shown as a bar diagram. Luciferase activity is expressed as mean relative to controls (n= 3). ***p<* 0.001. Student's t-test was used to calculate *p* value and error bars denote ± s.e.m.
Figure 2D is a drawing showing expression of miR-335 detected by *in situ* hybridization (left panels), on sections from normal healthy (non-AD affected) human skin (top panels) and AD lesional skin (bottom panels) (n=5). Immunohistochemical analysis of SOX6 (right panels), on sections from normal healthy human skin (top) and AD lesional skin (bottom). In unaffected skin, the expression of target gene remains relatively low (n= 5). However, a substantial increase in protein expression of target gene Sox6 is clearly observed in AD lesional skin (lower right) (n=5). In the upper panel basal layer is demarcated by dotted lines. Scale bars = 100 µm.

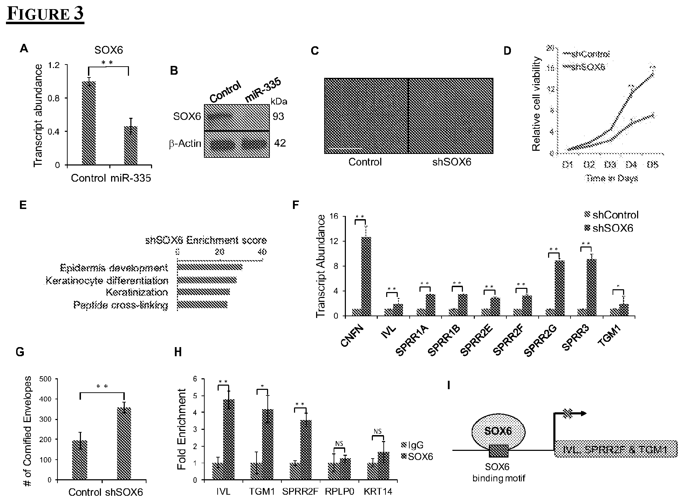

Figure 3A to Figure 3I are drawings showing targeting SOX6, miR-335 induces transcriptomic landscapes characteristic of epidermal differentiation
Figure 3A is a drawing showing a bar graph representing relative transcript abundance of SOX6 in N/TERT-1 cells transfected with miR-335 mimics or control cells.
Figure 3B is a drawing showing a Western blot showing the relative levels of SOX6 in control cells or cells transfected with miR-335 mimics. β-actin levels in the same samples indicate equal loading.
Figure 3C is a drawing showing representative phase-contrast images of N/TERT-1 cells expressing shRNA against SOX6 or scrambled control. Scale bar = 400 µm.
Figure 3D is a drawing showing relative number of cells in N/TERT-1 cells transduced with control shRNA and shSOX6 assessed by cell-titerglo luminescent assay.
Figure 3E is a drawing showing gene ontology analysis on differentially regulated genes upon SOX6 knockdown in N/TERT-1 keratinocytes.
Figure 3F is a drawing showing a bar graph which represents relative transcript abundance of factors associated with keratinocyte differentiation including IVL, TGM1 and SPRRs in N/TERT-1 keratinocytes transduced with control shRNA or shSOX6.
Figure 3G is a drawing showing bar graph which represents the number of cornified envelopes per field in N/TERT-1 keratinocytes transduced with shcontrol or shSOX6. A significant increase in number of CEs were observed upon SOX6 knock down. Values represent the mean of three independent experiments performed in quadruplicate, indicated by mean ± S.D. ** p-value < 0.01.
Figure 3H is a drawing showing a chromatin immunoprecipitation assay which was performed on N/TERT-1 keratinocytes using antibody specific for SOX6 or with a control IgG. Occupation of SOX6 on promoter regions of IVL, TGM1 and SPRR2F is shown as a fold enrichment over IgG. SOX6 enrichment was not seen in RPLP0 or KRT14 promoters.
Figure 3I is a drawing showing schematic depicting the SOX6 mediated transcriptional suppression of IVL, TGM1 and SPRR2F under basal conditions via a SOX6 binding motif in their promoters. Student's t-test was used to calculate *p* value and error bars denote ± s.e.m. **P<0.05 **P< 0.01.* NS- non-significant.

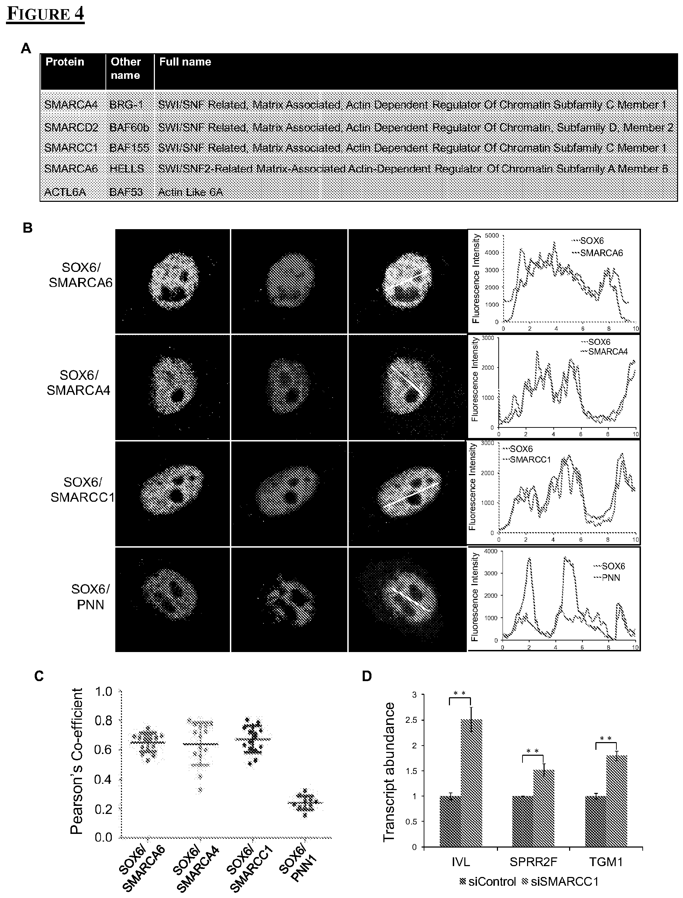

Figure 4A to Figure 4D are drawings showing that SOX6 interacts with SMARCA chromatin remodelling complex and stalls epidermal cell differentiation:
   Figure 4A is a drawing showing a list of selected, potential SOX6 interacting partners including subunits of SWI/SNF complex, detected by immunoprecipitation coupled mass spectrometry analysis.
   Figure 4B is a drawing showing that SOX6 co-localizes with SWI/SNF complex subunits. Immunocytochemistry on N/TERT-1 cells showing SOX6 (in green) co-stained with indicated subunit complexes (in red), namely, SMARCA6, SMARCA4 and SMARCC1. Z-stack images were acquired and a representative image on a single plane is shown. Right panel depicts relative fluorescent intensities along the white line. Pinin 1 (PNN1), a nuclear protein, did not show significant colocalization with SOX6.
   Figure 4C is a drawing showing that to quantify the extent of colocalization, Pearson colocalization co-efficient was calculated using Z-stack images with Olympus Flouview software. Scatter plot shows the Pearson colocalization coefficient obtained for each gene pair (SOX6 vs SMARCA6/SMARCA4/SMARCC1/PNN1) from ~ 15 nuclei from 2 independent experiments.
   Figure 4D is a drawing showing relative transcript abundance of IVL, SPRR2F and TGM1 mRNA levels in N/TERT-1 keratinocytes transiently transfected with a control siRNA or siRNA against SMARCC1. Student's t-test was used to calculate *p* value and error bars denote ± s.e.m. **P<0.05 **P<0.01.*

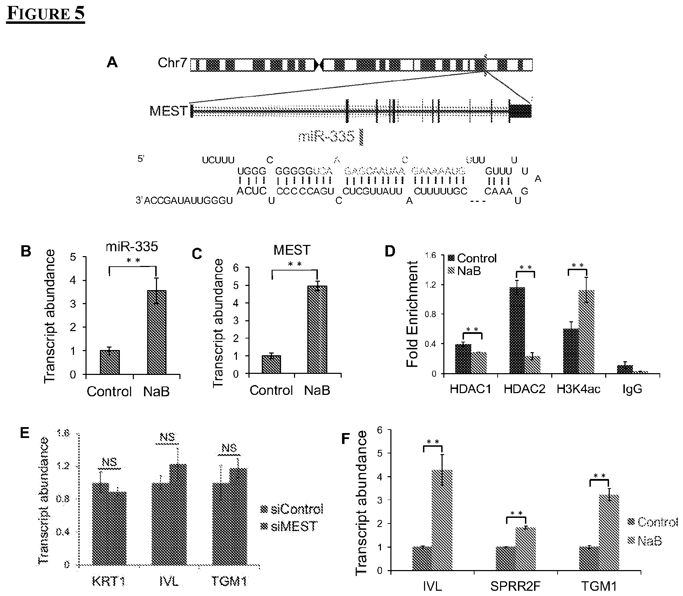

Figure 5A to Figure 5F are drawings showing epigenetic regulation of miR-335 by histone deacetylase 2 (HDAC2).
Figure 5A is a drawing showing the chromosomal location and genomic sequence of miR-335. miR-335 is located in the second intron of mesoderm-specific transcript (*MEST*) at the chromosome 7q32.2 locus. The stem loop sequence below indicates the precursor sequence of miR-335 and sequence (in green) specifies mature miR-335 sequence.
Figure 5B is a drawing showing a bar graph which represents relative expression of miR-335 in N/TERT-1 cells treated with sodium butyrate (NaB), analyzed by qRT- PCR. Ct values were normalized to U6 probe.
Figure 5C is a drawing showing relative expression of MEST in N/TERT-1 cells treated with sodium butyrate (NaB), analyzed by qRT- PCR. Ct values were normalized to U6 probe.
Figure 5D is a drawing showing chromatin immunoprecipitation (ChIP)-qPCR for HDAC1 and HDAC2 occupancy at the promoter region of miR-335 and MEST in N/TERT-1 in the presence of NaB. The precipitated DNA was analyzed by qRT- PCR. Data are expressed as means ± SD (n = 2).
Figure 5E is a drawing showing telative transcript abundance of keratinocyte differentiation markers *KRT1, IVL* and *TGM1* in N/TERT-1 cells upon knockdown of MEST represented as a bar graph.
Figure 5F is a drawing showing relative transcript abundance of IVL, SPRR2F and TGM1 in N/TERT-1 cells treated with NaB, as measured by qRT-PCR with Ct values were normalized to *RPLP0* probe. Data are representative of three independent experiments and plotted as mean ± S.D. Student's t-test was used to calculate *p* value * p-value < 0.05, ** p-value < 0.001.

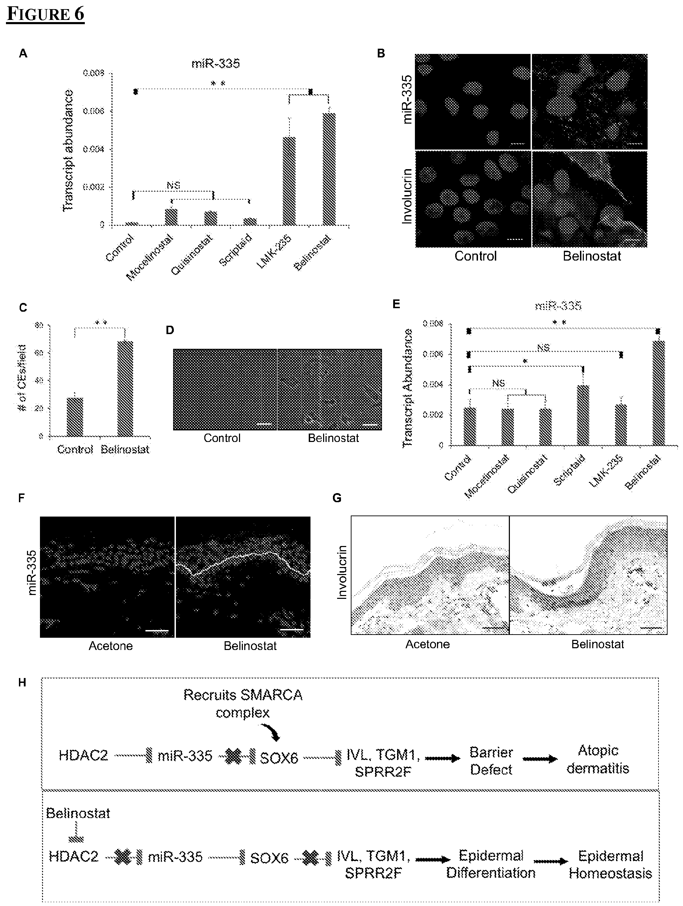

Figure 6A to Figure 6H are drawings showing that belinostat restores barrier function and epidermal homeostasis via miR-335 network
Figure 6A is a drawing showing qRT-PCR analysis of induced expression of miR-335 in N/TERT-1 cells upon treatment with HDAC inhibitors represented as a bar graph.
Figure 6B is a drawing showing induced expression of miR-335 upon treatment with belinostat, detected by *in situ* hybridization in N/TERT-1 cells using specific probes against mature miR-335 (red).
Figure 6C is a drawing showing bar graph representing the number of cornified envelopes per field in N/TERT-1 keratinocytes cells treated with belinostat.
Figure 6D is a drawing showing representative phase contrast microscopic image of mature cornified envelopes.
Figure 6E is a drawing showing qRT-PCR analysis of induced expression of miR-335 on human skin biopsies that were topically treated with acetone alone or HDACi dissolved in acetone, represented as a bar graph.
Figure 6F is a drawing showing expression of miR-335 in human skin biopsies that were topically treated with acetone alone or belinostat dissolved in acetone, detected by *in situ* hybridization using specific probes against mature miR-335 or control probe.
Figure 6G is a drawing showing immunohistochemical analysis of IVL, on sections from human skin biopsies that were topically treated with acetone alone or belinostat dissolved in acetone. Student's t-test was used to calculate *p* value * p-value < 0.05, ** p-value < 0.001. Scale bars, 100 µm.
Figure 6H is a drawing showing a model depicting how loss of miR-335 can lead to dysregulated molecular pathways in AD. Schematic representation of restoration of epidermal homeostasis upon treatment with belinostat.

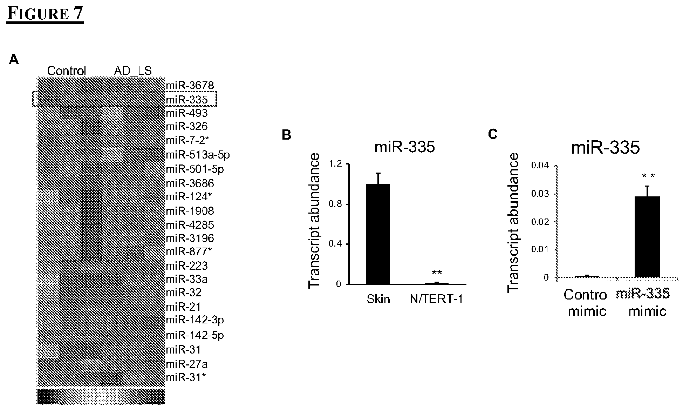

Figure 7A to Figure 7C are drawings showing differential expression of miR-335 in AD lesional skin
Figure 7A is a drawing showing a heat-map of selected genes generated from microRNA microarray using total RNA from AD lesional skin samples in comparison with RNA from normal human skin samples. Expression values displayed in shades of red (high) or blue (low) relative to the individual mean value of the gene in linear scale.
Figure 7B is a drawing showing relative transcript abundance of miR-335 in normal human skin versus N/TERT-1 keratinocyte cells, represented as a bar graph.
Figure 7C is a drawing showing relative transcript abundance of miR-335 in N/TERT-1 cells transfected with miR-335 mimics versus control cells, represented as a bar graph.

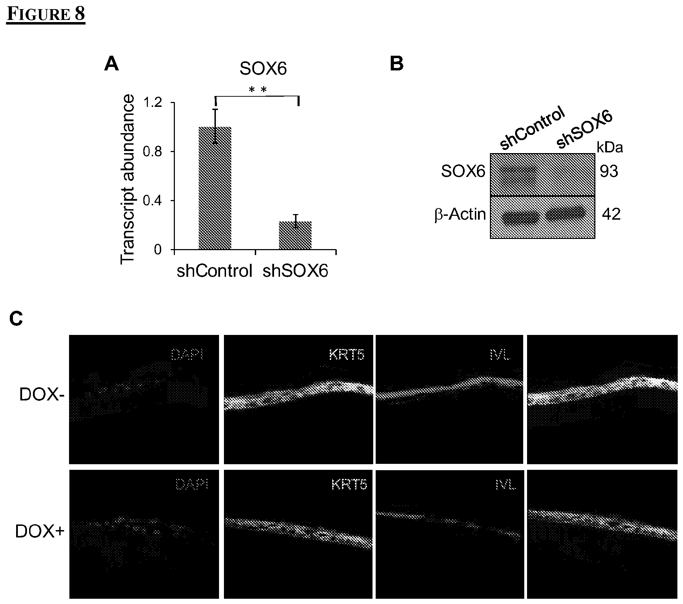

Figure 8A and Figure 8B are drawings showing SOX6 targets genes essential for keratinocyte differentiation and cornification
Figure 8A is a drawing showing relative transcript abundance of SOX6 in N/TERT-1 cells transduced with shSOX6 versus shControl, represented as a bar graph.
Figure 8B is a drawing showing a Western blot showing the relative levels of SOX6 in shSOX6 versus shControl. β-actin levels in the same samples indicate equal loading. C) Inducible expression of SOX6 leads to significant downregulation of IVL which is essential for keratinocyte differentiation and cornification as depicted in an organotypic assay.

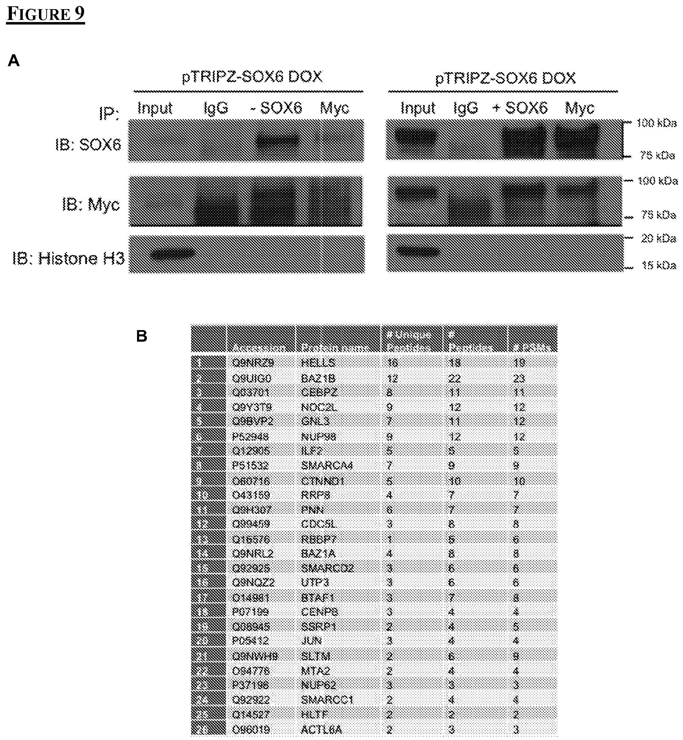

Figure 9A and Figure 9B are drawings showing that SOX6 interacts with the SMARCA complex
Figure 9A is a drawing showing immunoprecipitation of SOX6 protein from nuclear extracts of HEK293T cells transfected with pTRIPZ-SOX6 in the presence of doxycycline, followed by western blot analysis with indicated antibodies. Extracts were immunoprecipitated with rabbit IgG antibodies, antibodies to SOX6 or Myc.
Figure 9B is a drawing showing a list of potential SOX6 interacting partners.

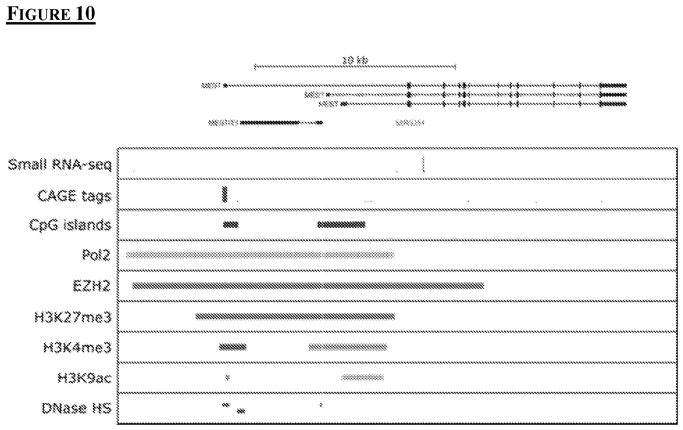

Figure 10 is a drawing showing promoter analysis of MEST/miR-335 locus. CAGE tags are only present in the upstream region of MEST, but not miR-335, suggesting that miR-335 does not have an independent promoter.

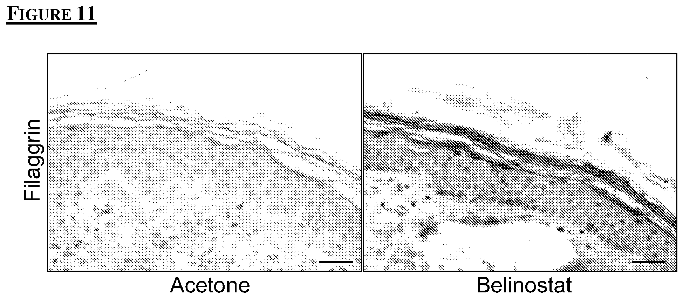

Figure 11 is a drawing showing that belinostat restores barrier function and FLG expression. Immunohistochemical analysis of FLG, on sections from human skin biopsies that were topically treated with acetone alone or Belinostat dissolved in acetone. Scale bars, 100 µm.

### DETAILED DESCRIPTION

Here, we present data from a screen for miRNAs specifically involved in atopic dermatitis. We identify miR-335 as essential for keratinocyte differentiation and maintenance of epidermal homeostasis.

We disclose the identification of the role of miR-335 in skin and atopic dermatitis.

Further we validate its loss of expression in AD lesional skin. We demonstrate that, in healthy skin, proliferating cells in the basal layer of the epidermis express low levels of miR-335, while high levels of miR-335 are expressed in suprabasal layers. We demonstrate that, in AD lesional skin, miR-335 expression is lost in all layers, including both basal and suprabasal layers.

We identify SOX6 as a direct target of miR-335. We demonstrate that, in the absence of miR-335, SOX6 recruits SMARCA complex components and causes epigenetic silencing of genes critical for epithelial differentiation, leading to the barrier defect.

Finally, we show that miR-335 is epigenetically regulated by histone deacetylases, and that treatment with the HDAC inhibitor Belinostat effectively restores miR-335 expression and epidermal homeostasis.

We therefore disclose the use of Belinostat to treat atopic dermatitis.

We propose the development of a topical cream with Belinostat to treat eczema.

### Abbreviations

AD-Atopic dermatitis, miR-335 -microRNA-335, 3'UTR- 3'-untranslated region, HDAC- histone deacetylases, LNA- locked nuclei acid, CE-cornified envelope, IVL-involucrin, SPRR- Small Proline Rich Proteins, TGM1-transglutaminase-1, ChIP -Chromatin immunoprecipitation and NaB -sodium butyrate.

### MIR-335

### MICRORNAs (MIRNAs)

microRNAs (miRNAs) are short (20-24 nt) non-coding RNAs that are involved in post-transcriptional regulation of gene expression in multicellular organisms by affecting both the stability and translation of mRNAs. miRNAs are transcribed by RNA polymerase II as part of capped and polyadenylated primary transcripts (pri-miRNAs) that can be either protein-coding or non-coding. The primary transcript is cleaved by the Drosha ribonuclease III enzyme to produce an approximately 70-nt stem-loop precursor miRNA (pre-miRNA), which is further cleaved by the cytoplasmic Dicer ribonuclease to generate the mature miRNA and antisense miRNA star (miRNA*) products. The mature miRNA is incorporated into a RNA-induced silencing complex (RISC), which recognizes target mRNAs through imperfect base pairing with the miRNA and most commonly results in translational inhibition or destabilization of the target mRNA. The encoded miRNA is dysregulated in a variety of cancers, including breast, colorectal, and prostate cancer.

### MIR-335

Reference to miR-335 includes reference to the sequence from any species, including cja-mir-335 (miRBASE accession number MI0031980); bta-mir-335 (miRBASE accession number MI0009804); ppy-mir-335 (miRBASE accession number MI0014900); efu-mir-335 (miRBASE accession number MI0028746); tch-mir-335 (miRBASE accession number MI0031291); dno-mir-335 (miRBASE accession number MI0039072); mmu-mir-335 (miRBASE accession number MI0000817); ssc-mir-335 (miRBASE accession number MI0013165); ocu-mir-335 (miRBASE accession number MI0039371); cfa-mir-335 (miRBASE accession number MI0008020); hsa-mir-335 (miRBASE accession number MI0000816); eca-mir-335 (miRBASE accession number MI0012696); chi-mir-335 (miRBASE accession number MI0030747); ptr-mir-335 (miRBASE accession number MI0008623); pal-mir-335 (miRBASE accession number MI0032526); ggo-mir-335 (miRBASE accession number MI0020669); rno-mir-335 (miRBASE accession number MI0000612); mml-mir-335 (miRBASE accession number MI0007699); cpo-mir-335 (miRBASE accession number MI0038733); hsa-miR-335-3p (miRBASE accession number MIMAT0004703); hsa-miR-335-5p (miRBASE accession number MIMAT0000765); mmu-miR-335-5p (miRBASE accession number MIMAT0000766); ggo-miR-335 (miRBASE accession number MIMAT0024122); dno-miR-335-5p (miRBASE accession number MIMAT0047815); mml-miR-335-3p (miRBASE accession number MIMAT0026853); eca-miR-335 (miRBASE accession number MIMAT0012942); cfa-miR-335 (miRBASE accession number MIMAT0006624); rno-miR-335 (miRBASE accession number MIMAT0000575); pal-miR-335-3p (miRBASE accession number MIMAT0040162); tch-miR-335-5p (miRBASE accession number MIMAT0036618); cja-miR-335-5p (miRBASE accession number MIMAT0039456); ocu-miR-335-3p (miRBASE accession number MIMAT0048383); bta-miR-335 (miRBASE accession number MIMAT0009291); efu-miR-335 (miRBASE accession number MIMAT0035059); cja-miR-335-3p (miRBASE accession number MIMAT0039457); pal-miR-335-5p (miRBASE accession number MIMAT0040161); mml-miR-335-5p (miRBASE accession number MIMAT0006274); cpo-miR-335-3p (miRBASE accession number MIMAT0047171); ocu-miR-335-5p (miRBASE accession number MIMAT0048382); ssc-miR-335 (miRBASE accession number MIMAT0013955); chi-miR-335-3p (miRBASE accession number MIMAT0036148); ppy-miR-335 (miRBASE accession number MIMAT0015837); cpo-miR-335-5p (miRBASE accession number MIMAT0047170); ptr-miR-335 (miRBASE accession number MIMAT0008104); mmu-miR-335-3p (miRBASE accession number MIMAT0004704); chi-miR-335-5p (miRBASE accession number MIMAT0036147); and dno-miR-335-3p (miRBASE accession number MIMAT0047816).

### HUMAN MIR-335

The human from of miR-335, hsa-mir-335 has miRBASE accession number MI0000816.
miR-335 has the following sequence:
   >hsa-mir-335 MI0000816 
UGUUUUGAGCGGGGUCAAGAGCAAUAACGAAAAAUGUUUGUCAUAAACCGUUUUUCA
UUAUUGCUCCUGACCUCCUCUCAUUUGCUAUAUUCA
hsa-mir-335 has the following structure:

    -------u  -uu    c        a         c        u  gu
5'          gu   ugag ggggguca gagcaauaa gaaaaaug uu  c
            ||   |||| |||||||| ||||||||| |||||||| ||
3'          cg   acuc ccuccagu cucguuauu cuuuuugc aa  a
    acuuauau  uuu    u        c         a        c  au

Mature hsa-mir-335 may comprise hsa-miR-335-5p or hsa-miR-335-3p.

Mature hsa-miR-335-5p has the miRBase accession number MIMAT0000765 and the following sequence:
>hsa-miR-335-5p MIMAT0000765
UCAAGAGCAAUAACGAAAAAUGU

Mature hsa-miR-335-3p has the miRBase accession number MIMAT0004703 and the following sequence:
>hsa-miR-335-3p MIMAT0004703
UUUUUCAUUAUUGCUCCUGACC

Unless the context dictates otherwise, the term miR-335 should be read as encompassing reference to any and all mature forms of miR-335.

With reference to hsa-mir-335, therefore, this term should, unless the context dictates otherwise, be taken to refer also to hsa-miR-335-5p and hsa-miR-335-3p, as the case may be.

hsa-mir-335 is described in detail in the following publications: "Identification of many microRNAs that copurify with polyribosomes in mammalian neurons" Kim J, Krichevsky A, Grad Y, Hayes GD, Kosik KS, Church GM, Ruvkun G, Proc Natl Acad Sci U S A. 101:360-365(2004), "New human and mouse microRNA genes found by homology search", Weber MJ, FEBS J. 272:59-73(2005), "A mammalian microRNA expression atlas based on small RNA library sequencing", Landgraf P, Rusu M, Sheridan R, Sewer A, Iovino N, Aravin A, Pfeffer S, Rice A, Kamphorst AO, Landthaler M, Lin C, Socci ND, Hermida L, Fulci V, Chiaretti S, Foa R, Schliwka J, Fuchs U, Novosel A, Muller RU, Schermer B, Bissels U, Inman J, Phan Q, Chien M, Cell. 129:1401-1414(2007) and "Patterns of known and novel small RNAs in human cervical cancer" Lui WO, Pourmand N, Patterson BK, Fire A, Cancer Res. 67:6031-6043(2007).

hsa-mir-335 is also described in detail in the following publications: Yu Y, et al. Sci Rep (2016) 6:30185, Zhang JK, et al. Cancer Cell Int (2017) 17:28, Shu M, et al. Mol Cancer (2011) 10:59, Jebbawi F, et al. J Transl Med (2014) 12:218, Zhou XM, et al. Oncotarget (2016) 7:13634-13650, Ronchetti D, et al. BMC Med Genomics (2008) 1:37, Samaraweera L, et al. BMC Cancer (2014) 14:309, Wang S, et al. BMC Ophthalmol (2018) 18:93, Huang HY, et al. PLoS One (2012) 7:e48637, Zarfeshani A, et al. Clin Epigenetics (2014) 6:27, Garcia-Cruz R, et al. BMC Med Genet (2015) 16:46, Vojtechova Z, et al. BMC Cancer (2016) 16:382, Rajpathak SN, et al. Sci Rep (2017) 7:43235, Murdocca M, et al. Int J Mol Sci (2016) 17, Maciotta S, et al. PLoS One (2012) 7:e43464, McAlinden A, et al. PLoS One (2013) 8:e75012, Schade A, et al. Int J Mol Sci (2013) 14:10710-10726, Lopez-Camarillo C, et al. Int J Mol Sci (2012) 13:1347-1379, Gassling V, et al. PLoS One (2013) 8:e63015, Zammit V, et al. Genes (Basel) (2018) 9, Wang G, et al. BMC Cancer (2017) 17:805, Cossellu G, et al. PLoS One (2016) 11:e0161916, Tang J, et al. Int J Mol Sci (2012) 13:13414-13437, Mainieri A, et al. Evol Med Public Health (2018) 2018:82-91, Allen-Rhoades W, et al. A, et al. Cancer Med (2015) 4:977-988, Lin CY, et al. Sci Rep (2018) 8:4277, Chen YJ, et al. Oncotarget (2017) 8:113598-113613, Shigunov P, et al. Sci Rep (2018) 8:8411, Liu Z, et al. Sci Rep (2016) 6:23709, Li R, et al. BMC Genomics (2015) 16:884, Hass R, et al. Cell Commun Signal (2012) 10:26, Tsai MM, et al. Int J Mol Sci (2016) 17, Shi C, et al. Oncotarget (2016) 7:40830-40845, Ekström K, et al. PLoS One (2013) 8:e75227, Melone MAB, et al. Cell Death Dis (2018) 9:228, Cava C, et al. BMC Syst Biol (2015) 9:62, Chan SH, et al. J Biomed Sci (2015) 22:9, Hanieh H et al Mol Cancer (2015) 14:172, Yeh CH, et al. Mol Cancer (2016) 15:37, Mulero-Navarro S, et al. Front Cell Dev Biol (2016) 4:45, Rodrigues CE, et al. L, et al. Stem Cell Res Ther (2017) 8:19, Jayavelu ND, et al. BMC Genomics (2015) 16:1077, Papanagnou P, et al. Biomolecules (2016) 6, Dahiya N, et al. PLoS One (2008) 3:e2436, Gupta S, et al. J Biomed Semantics (2016) 7:9, Gumerov V, et al. Biol Direct (2015) 10:59, Karere GM, et al. BMC Genomics (2012) 13:320, Wu HH, et al. Expert Rev Mol Med (2014) 16:e1, Panigrahi GK, et al. Oncotarget (2018) 9:13894-13910, Hossain MM, et al. J Ovarian Res (2013) 6:36, Watahiki A, et al. PLoS One (2011) 6:e24950, McGregor RA, et al. Curr Mol Med (2011) 11:304-316, Bettermann K, et al. Int J Mol Sci (2014) 15:9924-9944, Chen BS, et al. BMC Syst Biol (2016) 10:18, Yuan F, et al. Sci Rep (2018) 8:5674, Riester SM, et al. BMC Med Genomics (2015) 8:59, Li J, et al. BMC Genomics (2016) 17:517, Varshney J, et al. Front Mol Biosci (2015) 2:31, Bhattacharya A, et al. PLoS One (2012) 7:e46176, Xuan P, et al. PLoS One (2013) 8:e70204, Harries LW, Genes (Basel) (2014) 5:656-670, Hass R, et al. Cell Commun Signal (2011) 9:12, Huang Z, et al. Sci Rep (2017) 7:13673, Longati P, et al. BMC Cancer (2013) 13:95, Gurbuz I, et al. Mol Cancer (2014) 13:22, Natarajan SK, et al. Biomolecules (2015) 5:3309-3338, Pelosi L, et al. EBioMedicine (2015) 2:285-293, Sandhu SK, et al. Adv Hematol (2011) 2011:347137, Ren J, et al. J Transl Med (2018) 16:65, Koscianska E, et al. Cerebellum Ataxias (2014) 1:7, Zhan Y, et al. J Ovarian Res (2015) 8:48, Karere GM, et al. J Biomed Sci (2010) 17:54, Burba I, et al. PLoS One (2011) 6:e22158, Romania P, et al. Int J Mol Sci (2012) 13:16554-16579, Lopez-Anton M, et al. Biomed Res Int (2015) 2015:929806, Qin Z, et al. Viruses (2014) 6:4571-4580, Erriquez D, et al. Int J Mol Sci (2013) 14:19681-19704, Choi S, et al. Exp Mol Med (2017) 49:e403, Santhanam AN, et al. PLoS One (2009) 4:e4868, Ishii S, et al. Front Cell Neurosci (2015) 9:207, Zhu Z, et al. Viruses (2014) 6:1525-1539, Skalsky RL, et al. PLoS One (2011) 6:e24248, Sana J, et al. J Transl Med (2012) 10:103, Azuaje FJ, et al. BMC Med Genomics (2011) 4:59, Dalan AB, et al. BMC Cancer (2017) 17:207, Fiscon G, et al. Sci Rep (2018) 8:7769, Di Leva G, et al. Ups J Med Sci (2012) 117:202-216, Denk J, et al. PLoS One (2015) 10:e0126423, Sui J, et al. Oncotarget (2017) 8:65997-66018, Vuppalanchi R, et al. PLoS One (2013) 8:e74471, Zhang C, et al. Parasit Vectors (2016) 9:278, Xie S, et al. Sci Rep (2017) 7:2516, Koumangoye RB, et al. Mol Cancer (2015) 14:24, Jiao DM, et al. PLoS One (2017) 12:e0172470, Stigliani S, et al. Oncotarget (2015) 6:13295-13308, Chang L, et al. Oncotarget (2017) 8:84384-84395, Wang CH, et al. Oncotarget (2015) 6:42118-42129, Zhang ZJ, et al. Oncol Rep (2012) 27:903-910, Debeb BG, et al. Mol Cancer (2010) 9:180 and Valencia-Quintana R, et al. Front Microbiol (2014) 5:102.

### MIR-335 ACTIVITY

Biological activities of miR-335 are known in the art.

miR-335 activities and assays therefor are described for example in Kim et al (2015) miR-335 Targets SIAH2 and Confers Sensitivity to Anti-Cancer Drugs by Increasing the Expression of HDAC3. Mol Cells 38(6):562-72.

miR-335 activity may comprise effect on HDAC3 and/or SIAH2 expression. miR-335 activity may comprise increased sensitivity to anti-cancer drugs. miR-335 activity may comprise apoptotic effects. miR-335 activity may comprise inhibition of ubiquitination of HDAC3 in anti-cancer drug-resistant cancer cell lines. miR-335 activity may comprise negatively regulation of the invasion, migration, and growth rate of cancer cells. miR-335 activity may comprise negative regulation of the tumorigenic potential of cancer cells.

Other biological activities of miR-335 are described in the Examples.

miR-335 activity may comprise up-regulation of keratinocyte differentiation and/or cornification. Assays for such activity are set out in detail in the Examples.

miR-335 activity may comprise down-regulation of expression of *SOX6.* Such activity may be assayed by use of a luciferase reporter construct comprising the 3'-UTR of *SOX6.*

### MIR-335 MIRNAs

The methods and compositions described here may make use of miR-335, as well as variants, homologues, derivatives and fragments of any of these, for the diagnosis, detection of susceptibility to, treatment, alleviation or prophylaxis of a dermatological condition such as atopic dermatitis in an individual.

The terms "miR-335 miRNA" and "miR-335 nucleic acid" may be used interchangeably.

These terms are also intended to include a nucleic acid sequence capable of encoding an miR-335 miRNA and/or a fragment, derivative, homologue or variant of this. These terms are also intended to include a nucleic acid sequence which is a fragment, derivative, homologue or variant of an miR-335 polynucleotide having a specific sequence disclosed in this document.

Where reference is made to an miR-335 miRNA nucleic acid, this should be taken as a reference to a nucleic acid sequence capable of encoding such an miRNA. Such miRNAs may comprise one or more biological activities of a native miR-335, as the case may be.

miR-335 miRNAs may be used for a variety of means, as described in this document. For example, miR-335 miRNA may be used treat an individual suffering from, or suspected to be suffering from a dermatological condition such as atopic dermatitis, or to prevent such a condition or to alleviate any symptoms arising as a result of such a condition. Other uses will be evident to the skilled reader, and are also encompassed in this document.

The term "polynucleotide", as used in this document, generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

It will be understood by the skilled person that numerous nucleotide sequences can encode the same polypeptide as a result of the degeneracy of the genetic code.

As used herein, the term "nucleotide sequence" refers to nucleotide sequences, oligonucleotide sequences, polynucleotide sequences and variants, homologues, fragments and derivatives thereof (such as portions thereof). The nucleotide sequence may be DNA or RNA of genomic or synthetic or recombinant origin which may be double-stranded or single-stranded whether representing the sense or antisense strand or combinations thereof. The term nucleotide sequence may be prepared by use of recombinant DNA techniques (for example, recombinant DNA).

The term "nucleotide sequence" may mean DNA or RNA.

### Other Nucleic Acids

We also provide but do not claim nucleic acids which are fragments, homologues, variants or derivatives of miR-335 miRNA.

The terms "variant", "homologue", "derivative" or "fragment" in relation to miR-335 include any substitution of, variation of, modification of, replacement of, deletion of or addition of one (or more) nucleic acids from or to the sequence of an miR-335 miRNAs. Unless the context admits otherwise, references to "miR-335 miRNAs" and "miR-335 nucleic acid", "miR-335 nucleotide sequence" etc include references to such variants, homologues, derivatives and fragments of miR-335 miRNAs.

The nucleotide sequence may encode a polypeptide having any one or more miR-335 miRNA activity. The term "homologue" may be intended to cover identity with respect to structure and/or function such that the resultant nucleotide sequence encodes a polypeptide which has miR-335 miRNA activity. For example, a homologue etc of miR-335 miRNA may have an increased or decreased expression level in cells from an individual suffering from a dermatological condition such as atopic dermatitis compared to normal cells. With respect to sequence identity (i.e. similarity), there may be at least 70%, at least 75%, at least 85% or at least 90% sequence identity. There may be at least 95%, such as at least 98%, sequence identity to a relevant sequence such as any nucleic acid sequence of miR-335 miRNA. These terms also encompass allelic variations of the sequences.

The variant of miR-335 may comprise a sequence having 95% or more, 95.1% or more, 95.2% or more, 95.3% or more, 95.4% or more, 95.5% or more, 95.6% or more, 95.7% or more, 95.8% or more, 95.9% or more, 96% or more, 96.1% or more, 96.2% or more, 96.3% or more, 96.4% or more, 96.5% or more, 96.6% or more, 96.7% or more, 96.8% or more, 96.9% or more, 97% or more, 97.1% or more, 97.2% or more, 97.3% or more, 97.4% or more, 97.5% or more, 97.6% or more, 97.7% or more, 97.8% or more, 97.9% or more, 98% or more, 98.1% or more, 98.2% or more, 98.3% or more, 98.4% or more, 98.5% or more, 98.6% or more, 98.7% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more sequence identity thereto.

### Variants, Derivatives and Homologues

miR-335 miRNA nucleic acid variants, fragments, derivatives and homologues may comprise RNA. They may be single-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of this document, it is to be understood that the polynucleotides may be modified by any method available in the art. Such modifications may be carried out in order to enhance the *in vivo* activity or life span of polynucleotides of interest.

Where the polynucleotide is double-stranded, both strands of the duplex, either individually or in combination, are encompassed by the methods and compositions described here. Where the polynucleotide is single-stranded, it is to be understood that the complementary sequence of that polynucleotide is also included.

The terms "variant", "homologue" or "derivative" in relation to a nucleotide sequence include any substitution of, variation of, modification of, replacement of, deletion of or addition of one (or more) nucleic acid from or to the sequence. Said variant, homologues or derivatives may code for a polypeptide having biological activity. Such fragments, homologues, variants and derivatives of miR-335 may comprise modulated activity, as set out above.

As indicated above, with respect to sequence identity, a "homologue" may have at least 5% identity, at least 10% identity, at least 15% identity, at least 20% identity, at least 25% identity, at least 30% identity, at least 35% identity, at least 40% identity, at least 45% identity, at least 50% identity, at least 55% identity, at least 60% identity, at least 65% identity, at least 70% identity, at least 75% identity, at least 80% identity, at least 85% identity, at least 90% identity, or at least 95% identity to the relevant sequence, such as any nucleic acid sequence of a miR-335 miRNA.

There may be at least 95% identity, at least 96% identity, at least 97% identity, at least 98% identity or at least 99% identity.

The homologue, derivative or fragment of miR-335 may comprise a sequence having 95% or more, 95.1% or more, 95.2% or more, 95.3% or more, 95.4% or more, 95.5% or more, 95.6% or more, 95.7% or more, 95.8% or more, 95.9% or more, 96% or more, 96.1% or more, 96.2% or more, 96.3% or more, 96.4% or more, 96.5% or more, 96.6% or more, 96.7% or more, 96.8% or more, 96.9% or more, 97% or more, 97.1% or more, 97.2% or more, 97.3% or more, 97.4% or more, 97.5% or more, 97.6% or more, 97.7% or more, 97.8% or more, 97.9% or more, 98% or more, 98.1% or more, 98.2% or more, 98.3% or more, 98.4% or more, 98.5% or more, 98.6% or more, 98.7% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more sequence identity thereto.

Nucleotide identity comparisons may be conducted as described above. A sequence comparison program which may be used is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

### Hybridisation

We further describe nucleotide sequences that are capable of hybridising selectively to any of the sequences presented herein, or any variant, fragment or derivative thereof, or to the complement of any of the above. Nucleotide sequences may be at least 5, 10, or 15 nucleotides in length, such as at least 20, 30, 40 or 50 nucleotides in length.

The term "hybridization" as used herein shall include "the process by which a strand of nucleic acid joins with a complementary strand through base pairing" as well as the process of amplification as carried out in polymerase chain reaction technologies.

Polynucleotides capable of selectively hybridising to the nucleotide sequences presented herein, or to their complement, may be at least 40% homologous, at least 45% homologous, at least 50% homologous, at least 55% homologous, at least 60% homologous, at least 65% homologous, at least 70% homologous, at least 75% homologous, at least 80% homologous, at least 85% homologous, at least 90% homologous, or at least 95% homologous to the corresponding nucleotide sequences presented herein, such as any nucleic acid sequence of a miR-335 miRNA. Such polynucleotides may be generally at least 70%, at least 80 or 90% or at least 95% or 98% homologous to the corresponding nucleotide sequences over a region of at least 5, 10, 15 or 20, such as at least 25 or 30, for instance at least 40, 60 or 100 or more contiguous nucleotides.

The polynucleotide may comprise a sequence having 95% or more, 95.1% or more, 95.2% or more, 95.3% or more, 95.4% or more, 95.5% or more, 95.6% or more, 95.7% or more, 95.8% or more, 95.9% or more, 96% or more, 96.1% or more, 96.2% or more, 96.3% or more, 96.4% or more, 96.5% or more, 96.6% or more, 96.7% or more, 96.8% or more, 96.9% or more, 97% or more, 97.1% or more, 97.2% or more, 97.3% or more, 97.4% or more, 97.5% or more, 97.6% or more, 97.7% or more, 97.8% or more, 97.9% or more, 98% or more, 98.1% or more, 98.2% or more, 98.3% or more, 98.4% or more, 98.5% or more, 98.6% or more, 98.7% or more, 98.8% or more, 98.9% or more, 99% or more, 99.1% or more, 99.2% or more, 99.3% or more, 99.4% or more, 99.5% or more, 99.6% or more, 99.7% or more, 99.8% or more, or 99.9% or more sequence identity thereto.

The term "selectively hybridizable" means that the polynucleotide used as a probe is used under conditions where a target polynucleotide is found to hybridize to the probe at a level significantly above background. The background hybridization may occur because of other polynucleotides present, for example, in the cDNA or genomic DNA library being screened. In this event, background implies a level of signal generated by interaction between the probe and a non-specific DNA member of the library which is less than 10 fold, such as less than 100 fold as intense as the specific interaction observed with the target DNA. The intensity of interaction may be measured, for example, by radiolabelling the probe, e.g. with ³²P or ³³P or with non-radioactive probes (e.g., fluorescent dyes, biotin or digoxigenin).

Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained elsewhere in this document.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical polynucleotide sequences while an intermediate (or low) stringency hybridization can be used to identify or detect similar or related polynucleotide sequences.

We provide nucleotide sequences that may be able to hybridise to the miR-335nucleic acids, fragments, variants, homologues or derivatives under stringent conditions (e.g. 65°C and 0.1xSSC (1xSSC = 0.15 M NaCl, 0.015 M Na₃ Citrate pH 7.0)).

### Generation of Homologues, Variants and Derivatives

Polynucleotides which are not 100% identical to the relevant sequences (miR-335) but which are also included, as well as homologues, variants and derivatives of miR-335 miRNAs can be obtained in a number of ways. Other variants of the sequences may be obtained for example by probing RNA libraries made from a range of individuals, for example individuals from different populations.

For example, miR-335 miRNA homologues may be identified from other individuals, or other species. Examples of these are set out above.

Further recombinant miR-335 miRNA nucleic acids and polypeptides may be produced by identifying corresponding positions in the homologues, and synthesising or producing the molecule as described elsewhere in this document.

In addition, other viral/bacterial, or cellular homologues of miR-335 miRNAs, particularly cellular homologues found in mammalian cells (e.g. rat, mouse, bovine and primate cells), may be obtained and such homologues and fragments thereof in general will be capable of selectively hybridising to human miR-335 miRNAs. Such homologues may be used to design non-human miR-335 miRNA nucleic acids, fragments, variants and homologues. Mutagenesis may be carried out by means known in the art to produce further variety.

Sequences of miR-335 miRNA homologues may be obtained by probing libraries made from other animal species, and probing such libraries with probes comprising all or part of any of the miR-335 miRNA nucleic acids, fragments, variants and homologues, or other fragments of miR-335 miRNA under conditions of medium to high stringency.

Similar considerations apply to obtaining species homologues and allelic variants of the polypeptide or nucleotide sequences disclosed here.

Variants and strain/species homologues may also be obtained using degenerate PCR which will use primers designed to target sequences within the variants and homologues encoding conserved amino acid sequences within the sequences of the miR-335 miRNA nucleic acids. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences. It will be appreciated by the skilled person that overall nucleotide homology between sequences from distantly related organisms is likely to be very low and thus in these situations degenerate PCR may be the method of choice rather than screening libraries with labelled fragments the miR-335 sequences.

In addition, homologous sequences may be identified by searching nucleotide and/or protein databases using search algorithms such as the BLAST suite of programs.

Alternatively, such polynucleotides may be obtained by site directed mutagenesis of characterised sequences, for example, miR-335 miRNA nucleic acids, or variants, homologues, derivatives or fragments thereof. This may be useful where for example silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

The polynucleotides described here may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 8, 9, 10, or 15, such as at least 20, for example at least 25, 30 or 40 nucleotides in length, and are also encompassed by the term "polynucleotides" as used herein.

Polynucleotides such as a DNA polynucleotides and probes may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Primers comprising fragments of miR-335 miRNA are particularly useful in the methods of detection of miR-335 miRNA expression, such as up-regulation or down-regulation of miR-335 miRNA expression, for example, as associated with a dermatological condition such as atopic dermatitis. Suitable primers for amplification of miR-335 miRNA may be generated from any suitable stretch of miR-335 miRNA. Primers which may be used include those capable of amplifying a sequence of miR-335 miRNA which is specific.

Although miR-335 miRNA primers may be provided on their own, they are most usefully provided as primer pairs, comprising a forward primer and a reverse primer.

Longer polynucleotides will generally be produced using recombinant means, for example using a PCR (polymerase chain reaction) cloning techniques. This will involve making a pair of primers (e.g. of about 15 to 30 nucleotides), bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

Polynucleotides or primers may carry a revealing label. Suitable labels include radioisotopes such as ³²P or ³⁵S, digoxigenin, fluorescent dyes, enzyme labels, or other protein labels such as biotin. Such labels may be added to polynucleotides or primers and may be detected using by techniques known *per se.* Polynucleotides or primers or fragments thereof labelled or unlabeled may be used by a person skilled in the art in nucleic acid-based tests for detecting or sequencing polynucleotides in the human or animal body.

Such tests for detecting generally comprise bringing a biological sample containing DNA or RNA into contact with a probe comprising a polynucleotide or primer under hybridising conditions and detecting any duplex formed between the probe and nucleic acid in the sample. Such detection may be achieved using techniques such as PCR or by immobilising the probe on a solid support, removing nucleic acid in the sample which is not hybridised to the probe, and then detecting nucleic acid which has hybridised to the probe. Alternatively, the sample nucleic acid may be immobilised on a solid support, and the amount of probe bound to such a support can be detected. Suitable assay methods of this and other formats can be found in for example WO89/03891 and WO90/13667.

Tests for sequencing nucleotides, for example, the miR-335 miRNA nucleic acids, involve bringing a biological sample containing target DNA or RNA into contact with a probe comprising a polynucleotide or primer under hybridising conditions and determining the sequence by, for example the Sanger dideoxy chain termination method (see Sambrook *et al.).*

Such a method generally comprises elongating, in the presence of suitable reagents, the primer by synthesis of a strand complementary to the target DNA or RNA and selectively terminating the elongation reaction at one or more of an A, C, G or T/U residue; allowing strand elongation and termination reaction to occur; separating out according to size the elongated products to determine the sequence of the nucleotides at which selective termination has occurred. Suitable reagents include a DNA polymerase enzyme, the deoxynucleotides dATP, dCTP, dGTP and dTTP, a buffer and ATP. Dideoxynucleotides are used for selective termination.

### ISOLATION OF MIRNAs

miRNAs may be isolated from exosomes using any means known in the art.

The person skilled in the art will be aware of the various methods for isolation of miRNAs from biological fluids that have been developed. Commercially available miRNA isolation kits are available, for example from miRNeasy kit (Qiagen, CA), the miRVana PARIS kit (Ambion, TX), and the total RNA isolation kit (Norgen Biotek, Canada). Any of these may be used to isolate miRNAs from a sample.

The following example protocol, from the miRNeasy Serum/Plasma Handbook (QIAGEN, February 2012), may be used to isolate miRNA using the miRNeasy kit:
1. Prepare serum or plasma or thaw frozen samples.
2. Add 5 volumes QIAzol Lysis Reagent (see Table 2 for guidelines). Mix by vortexing or pipetting up and down.

| **Serum/plasma (µl)** | **Protocol step 2: QIAzol Lysis Reagent (µl)** | **Protocol step 5: chloroform (µl)** | **Protocol step 7: approx.. volume of upper aqueous phase (µl)** | **Protocol step 8: 100% ethanol (µl)** |
|---|---|---|---|---|
| ≤ 50 | 250 | 50 | 150 | 225 |
| 100 | 500 | 100 | 300 | 450 |
| 200 | 1000 | 200 | 600 | 900 |

| | | | | |
|---|---|---|---|---|
| Note: If the volume of plasma or serum is not limited, we recommend using 100-200 µl per RNA preparation. Note: After addition of QIAzol Lysis Reagent, lysates can be stored at -70oC for several months. | | | | |

3. Place the tube containing the lysate on the benchtop at room temperature (15-25°C) for 5 min.
4. Add 3.5 µl miRNeasy Serum/Plasma Spike-In Control (1.6 x 10⁸ copies/µl working solution) and mix thoroughly.
For details on making appropriate stocks and working solutions of miRNeasy Serum/Plasma Spike-In Control, see Appendix B, page 25
5. Add chloroform of an equal volume to the starting sample to the tube containing the lysate and cap it securely (see Table 2 for guidelines). Vortex or shake vigorously for 15 s.
Thorough mixing is important for subsequent phase separation.
6. Place the tube containing the lysate on the benchtop at room temperature (15-25°C) for 2-3 min.
7. Centrifuge for 15 min at 12,000 x g at 4°C. After centrifugation, heat the centrifuge up to room temperature (15-25°C) if the same centrifuge will be used for the next centrifugation steps.
After centrifugation, the sample separates into 3 phases: an upper, colorless, aqueous phase containing RNA; a white interphase; and a lower, red, organic phase. See Table 2 for the approximate volume of the aqueous phase.
8. Transfer the upper aqueous phase to a new collection tube (not supplied). Avoid transfer of any interphase material. Add 1.5 volumes of 100% ethanol and mix thoroughly by pipetting up and down several times. Do not centrifuge. Continue without delay with step 9.
A precipitate may form after addition of ethanol, but this will not affect the procedure.
9. Pipet up to 700 µl of the sample, including any precipitate that may have formed, into an RNeasy MinElute spin column in a 2 ml collection tube (supplied). Close the lid gently and centrifuge at ≥8000 x g (≥10,000 rpm) for 15 s at room temperature (15-25°C). Discard the flow-through. *
Reuse the collection tube in step 10.
10. Repeat step 9 using the remainder of the sample. Discard the flow- through. *
Reuse the collection tube in step 11.
11. Add 700 µl Buffer RWT to the RNeasy MinElute spin column. Close the lid gently and centrifuge for 15 s at ≥8000 x g (≥10,000 rpm) to wash the column. Discard the flow-through. *
Reuse the collection tube in step 12.
12. Pipet 500 µl Buffer RPE onto the RNeasy MinElute spin column. Close the lid gently and centrifuge for 15 s at ≥8000 x g (≥10,000 rpm) to wash the column. Discard the flow-through.
Reuse the collection tube in step 13.
13. Pipet 500 µl of 80% ethanol onto the RNeasy MinElute spin column. Close the lid gently and centrifuge for 2 min at ≥8000 x g (≥10,000 rpm) to wash the spin column membrane. Discard the collection tube with the flow-through.
Note: 80% ethanol should be prepared with ethanol (96-100%) and RNase-free water.
Note: After centrifugation, carefully remove the RNeasy MinElute spin column from the collection tube so that the column does not contact the flow-through. Otherwise, carryover of ethanol will occur.

14. Place the RNeasy MinElute spin column into a new 2 ml collection tube (supplied). Open the lid of the spin column, and centrifuge at full speed for 5 min to dry the membrane. Discard the collection tube with the flow-through.

To avoid damage to their lids, place the spin columns into the centrifuge with at least one empty position between columns. Orient the lids so that they point in a direction opposite to the rotation of the rotor (e.g., if the rotor rotates clockwise, orient the lids counterclockwise).

It is important to dry the spin column membrane, since residual ethanol may interfere with downstream reactions. Centrifugation with the lids open ensures that no ethanol is carried over during RNA elution.

15. Place the RNeasy MinElute spin column in a new 1.5 ml collection tube (supplied). Add 14 µl RNase-free water directly to the center of the spin column membrane. Close the lid gently, and centrifuge for 1 min at full speed to elute the RNA.

As little as 10 µl RNase-free water can be used for elution if a higher RNA concentration is required, but the yield will be reduced by approximately 20%. Do not elute with less than 10 µl RNase-free water, as the spin column membrane will not be sufficiently hydrated.

The dead volume of the RNeasy MinElute spin column is 2 µl : elution with 14 µl RNase-free water results in a 12 µl eluate.

### HISTONE DEACETYLASE INHIBITORS (HDAC INHIBITORS - HDIS)

We disclose but do not claim the use of an histone deacetylase inhibitor (HDAC inhibitor) in a method of diagnosis, treatment, prophylaxis or alleviation of a dermatological condition such as atopic dermatitis. HDAC inhibitors may be known by various names, such as lysine deacetylases (KDAC).

The HDAC inhibitor may be anything that is capable of reducing any one or more of the activities of a histone deacetylase. Such an activity may include the deacetylase activity of a histone. The HDAC inhibitor may inhibit the removal of an acetyl group an ε-N-acetyl lysine amino acid on a histone by histone deacetylase. Assays for deacetylase activity are known in the art.

HDAC proteins are grouped into four classes based on function and DNA sequence similarity:
- Class I, which includes HDAC1, -2, -3 and -8 are related to yeast RPD3 gene;
- Class IIA, which includes HDAC4, -5, -7 and -9; Class IIB -6, and -10 are related to yeast Hda1 gene;
- Class III, also known as the sirtuins are related to the Sir2 gene and include SIRT1-7
- Class IV, which contains only HDAC11 has features of both Class I and II.

Class I, II and IV are considered "classical" HDACs whose activities are inhibited by trichostatin A (TSA) and have a zinc dependent active site, whereas Class III enzymes are a family of NAD+-dependent proteins known as sirtuins and are not affected by TSA. Homologues to these three groups are found in yeast having the names: reduced potassium dependency 3 (Rpd3), which corresponds to Class I; histone deacetylase 1 (hda1), corresponding to Class II; and silent information regulator 2 (Sir2), corresponding to Class III. Class IV contains just one isoform (HDAC11), which is not highly homologous with either Rpd3 or hda1 yeast enzymes, and therefore HDAC11 is assigned to its own class. The Class III enzymes are considered a separate type of enzyme and have a different mechanism of action; these enzymes are NAD+-dependent, whereas HDACs in other classes require Zn2+ as a cofactor.

The HDAC inhibitors described here may inhibit an activity of any of the classes of histones set out above. HDAC inhibitors themselves may be classified into a number of groups, including:
- hydroxamic acids (or hydroxamates), such as trichostatin A,
- cyclic tetrapeptides (such as trapoxin B), and the depsipeptides,
- benzamides,
- electrophilic ketones, and
- aliphatic acid compounds such as phenylbutyrate and valproic acid.

"Second-generation" HDIs include the hydroxamic acids vorinostat (SAHA), belinostat (PXD101), LAQ824, and panobinostat (LBH589); and the benzamides : entinostat (MS-275), tacedinaline (CI994), and mocetinostat (MGCD0103).

Any of these HDAC inhibitors may be used for the purposes described in this document.

### MOCETINOSTAT

Mocetinostat has PubChem CID 9865515 and is also known as 726169-73-9, MGCD0103, MGCD-0103 and N-(2-AMINOPHENYL)-4-([[4-(PYRIDIN-3-YL)PYRIMIDIN-2-YL]AMINO]METHYL)BENZAMIDE.

Mocetinostat is a rationally designed, orally available, Class 1-selective, small molecule, 2-aminobenzamide HDAC inhibitor with potential antineoplastic activity. Mocetinostat binds to and inhibits Class 1 isoforms of HDAC, specifically HDAC 1, 2 and 3, which may result in epigenetic changes in tumor cells and so tumor cell death; although the exact mechanism has yet to be defined, tumor cell death may occur through the induction of apoptosis, differentiation, cell cycle arrest, inhibition of DNA repair, upregulation of tumor suppressors, down regulation of growth factors, oxidative stress, and autophagy, among others. Overexpression of Class I HDACs 1, 2 and 3 has been found in many tumors and has been correlated with a poor prognosis.

### QUISINOSTAT

Quisinostat has PubChem CID 11538455 and is also known as 875320-29-9, JNJ-26481585, N-Hydroxy-2-(4-((((1-methyl-1H-indol-3-yl)methyl)amino)methyl)piperidin-1-yl)pyrimidine-5-carboxamide and UNII-9BJ85K1J8S.

Quisinostat is an orally bioavailable, second-generation, hydroxamic acid-based inhibitor of histone deacetylase (HDAC) with potential antineoplastic activity. HDAC inhibitor JNJ-26481585 inhibits HDAC leading to an accumulation of highly acetylated histones, which may result in an induction of chromatin remodeling; inhibition of the transcription of tumor suppressor genes; inhibition of tumor cell division; and the induction of tumor cell apoptosis. HDAC, an enzyme upregulated in many tumor types, deacetylates chromatin histone proteins. Compared to some first generation HDAC inhibitors, JNJ-26481585 may induce superior HSP70 upregulation and bcl-2 downregulation.

### SCRIPTAID

Scriptaid has PubChem CID 5186 and is also known as 287383-59-9, Scriptide, 6-(1,3-Dioxo-1H-benzo[de]isoquinolin-2(3H)-yl)-N-hydroxyhexanamide and GCK 1026.

### LMK-235

LMK-235 has PubChem CID 71520717 and is also known as 1418033-25-6, LMK 235, N-((6-(hydroxyamino)-6-oxohexyl)oxy)-3,5-dimethylbenzamide and CHEMBL2312168.

### BELINOSTAT

Belinostat has PubChem CID 6918638 and a molecular formula of C₁₅H₁₄N₂O₄S.

Belinostat is also known by the names CID 6918638, Belinostat, 414864-00-9, PXD101, Belinostat (PXD101), 866323-14-0, PXD-101, Beleodaq, (E)-N-hydroxy-3-(3-(N-phenylsulfamoyl)phenyl)acrylamide, NSC726630, PXD 101, N-HYDROXY-3-(3-PHENYLSULFAMOYLPHENYL)ACRYLAMIDE, UNII-F4H96P17NZ, N-HYDROXY-3-[3-[(PHENYLAMINO)SULFONYL]PHENYL]-2-PROPENAMIDE, PX-105684, 2-Propenamide, N-hydroxy-3-[3-[(phenylamino)sulfonyl]phenyl]-, (2E)-, F4H96P17NZ, (2E)-N-hydroxy-3-[3-(phenylsulfamoyl)phenyl]prop-2-enamide, CHEBI:61076, PX 105684, (2E)-N-hydroxy-3-[3-(phenylsulfamoyl)phenyl]acrylamide, (E)-N-hydroxy-3-[3-(phenylsulfamoyl)phenyl]prop-2-enamide., E-Belinostat, (E)-N-hydroxy-3-[3-(phenylsulfamoyl)phenyl]prop-2-enamide, Belinostat [USAN:INN], Belinostat(Random Configuration), N-hydroxy-3-(3-(phenylsulfamoyl)phenyl)prop-2-enamide, PX105684, 2-Propenamide, N-hydroxy-3-(3-((phenylamino)sulfonyl)phenyl)-, (2E)-, Belinostat Ph3, Beleodaq (TN), PubChem22405, Belinostat - PXD101, Belinostat (USAN/INN), N-Hydroxy-3-(3-phenylsulphamoylphenyl)acrylamide, cc-489, MLS006011091, CHEMBL408513, GTPL7496, Belinostat 866323-14-0, BDBM25150, CHEBI:94531, DTXSID60194378, EX-A180, QCR-181, (E)-3-[3-(phenylsulfamoyl)phenyl]prop-2-enehydroxamic acid, BCPP000351, AOB87787, BCP01741, ZINC3818726, Belinostat,PXD101, PX105684, Belinostat/PXD101,PX105684/, ABP000140, s1085, AKOS025401741, BCP9000386, CCG-208758, DB05015, LS41098, NSC-726630, SB16466, NCGC00263155-05, AC-25046, AS-17068, SC-71101, SMR004702879, AB0007889, SW219445-1, EC-000.2286, A25012, D08870, W-5363, J-523584, Q4882925, BRD-K17743125-001-01-9, N-Hydroxy-3-[(phenylamino)sulfonyl]-trans-cinnamamide, (E)-N-Hydroxy-3-(3-phenylsulfamoyl-phenyl)-acrylamide, N-HYDROXY-3-[3-[(PHENYLAMINO)SULFONYL]PHENYL]-2-PR and 50G.

Belinostat is a novel hydroxamic acid-type histone deacetylase (HDAC) inhibitor with antineoplastic activity. Belinostat targets HDAC enzymes, thereby inhibiting tumor cell proliferation, inducing apoptosis, promoting cellular differentiation, and inhibiting angiogenesis. This agent may sensitize drug-resistant tumor cells to other antineoplastic agents, possibly through a mechanism involving the down-regulation of thymidylate synthase (National Cancer Institute).

Drug screening lead to the identification of "Belinostat", which is a broad spectrum HDAC inhibitor as a candidate drug.

Belinostat can effectively restore miR-335 expression, suppress pro-inflammatory factors and repair the defective barrier, thus alleviating the therapeutically intractable a dermatological condition such as atopic dermatitis.

Belinostat may be used in its native from, or as a salt, hydrate, or solvate. It may therefore be convenient or desirable to prepare, purify, and/or handle a corresponding salt of belinostat, for example, a pharmaceutically-acceptable salt. Examples of pharmaceutically acceptable salts are discussed in Berge et at., 1977, "Pharmaceutically Acceptable Salts" J. Pharm. ScL. Vol. 66, pp. 1-19.

Examples of suitable inorganic cations include, but are not limited to, alkali metal ions such as Na+ and K+, alkaline earth cations such as Ca²⁺ and Mg²⁺, and other cations such as Al⁺³. Examples of suitable organic cations include, but are not limited to, ammonium ion (i.e., NH⁴⁺) and substituted ammonium ions (e.g., NH₃R⁺, NH₂R²⁺, NHR³⁺, NR⁴⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)⁴⁺.

Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose.

It may be convenient or desirable to prepare, purify, and/or handle a corresponding solvate of belinostat. The term "solvate" is used herein in the conventional sense to refer to a complex of solute (e.g., belinostat, salt of belinostat) and solvent. If the solvent is water, the solvate may be conveniently referred to as a hydrate, for example, a mono-hydrate, a dihydrate, a tri-hydrate, etc.

Where reference is made to belinostat in this document, it should be read as a reference also to a salt, hydrate, or solvate thereof.

### BELINOSTAT ANALOGUES

Analogues and derivatives of belinostat are known in the art. Such analogues and derivatives preferably comprise histone deacetylase (HDAC) inhibitor activity. They may also comprise a N-hydroxycinnamamide moiety.

For example, analogues and derivatives of belinostat are described in Zhang et al (2019) Design, synthesis and evaluation of belinostat analogs as histone deacetylase inhibitors. Future Medicinal Chemistry, 11(21) and Li et al (2019) Design, synthesis, and biological evaluation of target water-soluble hydroxamic acid-based HDACi derivatives as prodrugs. Chemical Biology & Drug Design 94(4), 1760-1767.

An example of a belinostat analogue/derivative is compound 7e, described in Zhang et al (2019), which has been shown to exhibit an IC₅₀ value of 11.5 nM in an HDAC inhibition assay.

Unless the context dictates otherwise, where reference is made to belinostat, this should include reference to analogues and derivatives of belinostat.

### DERMATOLOGICAL CONDITIONS

We disclose the use of miR-335, an agent capable of up-regulating the expression or activity of miR-335, a histone deacetylase (HDAC) inhibitor or belinostat, for the treatment, prophylaxis, prevention or alleviation of a dermatological disease or condition.

Examples of dermatological diseases and conditions are known in the art and include for example alopecia areata, atopic dermatitis, bullous pemphigoid, bullous systemic lupus erythematosus, dermatitis herpetiformis (DH), dermatomyositis, drug-induced pemphigus, eczema, epidermolysis bullosa acquisita (EBA), IgA pemphigus, lichen sclerosus, linear IgA bullous dermatosis, mucous membrane pemphigoid, paraneoplastic pemphigus, pemphigoid gestationis, pemphigus, pemphigus erythematosus (PE), pemphigus foliaceus, pemphigus vegetans, pemphigus vulgaris, psoriasis, scleroderma, systemic sclerosis and vitiligo.

The dermatological disease or condition may comprise for example atopic dermatitis (eczema).

### ATOPIC DERMATITIS (AD) OR ECZEMA

Atopic dermatitis (AD) is a chronic relapsing inflammatory skin condition, whereby barrier defect and exposure to allergens initiate development of the disease.

Defective skin barrier and exposure to allergens, contribute to disease initiation and development.

Atopic dermatitis (eczema) is a condition that makes skin red and itchy. Atopic dermatitis is common in children but can occur at any age. Atopic dermatitis is long lasting (chronic) and tends to flare periodically. It may be accompanied by asthma or hay fever.

Atopic dermatitis (eczema) signs and symptoms vary widely from person to person and include: dry skin, itching, which may be severe, especially at night, red to brownish-gray patches, especially on the hands, feet, ankles, wrists, neck, upper chest, eyelids, inside the bend of the elbows and knees, and in infants, the face and scalp, small, raised bumps, which may leak fluid and crust over when scratched, thickened, cracked, scaly skin and raw, sensitive, swollen skin from scratching.

Atopic dermatitis most often begins before age 5 and may persist into adolescence and adulthood. For some people, it flares periodically and then clears up for a time, even for several years.

### PHARMACEUTICAL COMPOSITIONS

We disclose pharmaceutical compositions comprising miR-335, an agent capable of up-regulating the expression or activity of miR-335, a histone deacetylase (HDAC) inhibitor or belinostat.

While it is possible for the composition comprising the miR-335, an agent capable of up-regulating the expression or activity of miR-335, a histone deacetylase (HDAC) inhibitor or belinostat to be administered alone, it is preferable to formulate the active ingredient as a pharmaceutical formulation.

The pharmaceutical formulations disclosed here comprise an effective amount of miR-335, an agent capable of up-regulating the expression or activity of miR-335, a histone deacetylase (HDAC) inhibitor or belinostat together with one or more pharmaceutically-acceptable carriers.

An "effective amount" of miR-335, an agent capable of up-regulating the expression or activity of miR-335, a histone deacetylase (HDAC) inhibitor or belinostat is the amount sufficient to restore, maintain or enhance skin barrier function in an individual.

The effective amount will vary depending upon the particular disease or syndrome to be treated or alleviated, as well as other factors including the age and weight of the patient, how advanced the disease etc state is, the general health of the patient, the severity of the symptoms, and whether the miR-335, an agent capable of up-regulating the expression or activity of miR-335, a histone deacetylase (HDAC) inhibitor or belinostat is being administered alone or in combination with other therapies.

The term "treatment" therefore includes combination treatments and therapies, in which two or more treatments or therapies are combined, for example, sequentially or simultaneously. For example, belinostat may also be used in combination therapies, e.g., in conjunction with other agents, for example, dermatological, etc. Examples of treatments and therapies include, but are not limited to, chemotherapy (the administration of active agents, including, e.g., HDAC inhibitors, antibodies (e.g., as in immunotherapy), prodrugs (e.g., as in photodynamic therapy, GDEPT, ADEPT, etc.); surgery; radiation therapy; and gene therapy.

The miR-335, a histone deacetylase (HDAC) inhibitor or belinostat may be applied in any suitable quantity. For example, a composition containing 10µg or less, such as 5µg or less, such as 2µg or less, such as 1µg or less, such as 0.5µg or less, such as 0.3 µg of miR-335, a histone deacetylase (HDAC) inhibitor or belinostat may be applied to subject.

The pharmaceutical composition may comprise 40 µg/ml or less, 20 µg/ml or less, 8 µg/ml or less, 4 µg/ml or less, 2 µg/ml or less or 1.2 µg/ml or less of miR-335, a histone deacetylase (HDAC) inhibitor or belinostat.

The composition may be administered for any suitable length of time, such as at least one week to twelve weeks. The amount of miR-335, a histone deacetylase (HDAC) inhibitor or belinostat that is administered may comprise any suitable amount, such as about 0.0001 milligram to about 100 g per day.

An effective amount of a pharmaceutical composition described here may comprise any amount that is effective to achieve its purpose. The effective amount, usually expressed in mg/kg can be determined by routine methods during pre-clinical and clinical trials by those of skill in the art.

The miR-335, a histone deacetylase (HDAC) inhibitor or belinostat may be administered to an animal such as a mammal in need thereof. The animal may be any animal. Examples include a laboratory animal, such as a mouse, rat, or guinea pig; or a primate, such as a monkey, orangutan, ape, chimpanzee, or human. For example, the mammal may be a human.

Suitable pharmaceutically acceptable carriers are well known in the art and vary with the desired form and mode of administration of the pharmaceutical formulation. For example, they may include diluents or excipients such as fillers, binders, wetting agents, disintegrators, surface-active agents, lubricants and the like. Typically, the carrier is a solid, a liquid or a vaporizable carrier, or a combination thereof. Each carrier should be "acceptable" in the sense of being compatible with the other ingredients in the formulation and not injurious to the patient. The carrier should be biologically acceptable without eliciting an adverse reaction (e.g. immune response) when administered to the host.

The pharmaceutical compositions include topical formulations which are preferred where the tissue affected is primarily the skin or epidermis (for example, epidermal diseases such as atopic dermatitis, etc). The topical formulations include those pharmaceutical forms in which the composition is applied externally by direct contact with the skin surface to be treated. A conventional pharmaceutical form for topical application includes a soak, an ointment, a cream, a lotion, a paste, a gel, a stick, a spray, an aerosol, a bath oil, a solution and the like. Topical therapy is delivered by various vehicles, the choice of vehicle can be important and generally is related to whether an acute or chronic disease is to be treated.

Lotions (powder in water suspension) and solutions (medications dissolved in a solvent) are ideal for hairy and intertriginous areas. Ointments or water-in-oil emulsions, are the most effective hydrating agents, appropriate for dry scaly eruptions, but are greasy and depending upon the site of the lesion sometimes undesirable.

As appropriate, they can be applied in combination with a bandage, particularly when it is desirable to increase penetration of the miR-335, an agent capable of up-regulating the expression or activity of miR-335, a histone deacetylase (HDAC) inhibitor or belinostat composition into a lesion. Creams or oil-in-water emulsions and gels are absorbable and are the most cosmetically acceptable to the patient. (Guzzo et al, in Goodman & Gilman's Pharmacological Basis of Therapeutics, 9th Ed., p. 1593-15950 (1996)). Cream formulations generally include components such as petroleum, lanolin, polyethylene glycols, mineral oil, glycerin, isopropyl palmitate, glyceryl stearate, cetearyl alcohol, tocopheryl acetate, isopropyl myristate, lanolin alcohol, simethicone, carbomen, methylchlorisothiazolinone, methylisothiazolinone, cyclomethicone and hydroxypropyl methylcellulose, as well as mixtures thereof.

Other formulations for topical application include shampoos, soaps, shake lotions, and the like, particularly those formulated to leave a residue on the underlying skin, such as the scalp (Arndt et al, in Dermatology In General Medicine 2:2838 (1993)).

In general, the concentration of the miR-335, an agent capable of up-regulating the expression or activity of miR-335, a histone deacetylase (HDAC) inhibitor or belinostat composition in the topical formulation is in an amount of about 0.5 to 50% by weight of the composition, preferably about 1 to 30%, more preferably about 2-20%, and most preferably about 5-10%. The concentration used can be in the upper portion of the range initially, as treatment continues, the concentration can be lowered or the application of the formulation may be less frequent. Topical applications are often applied twice daily. However, once-daily application of a larger dose or more frequent applications of a smaller dose may be effective. The stratum corneum may act as a reservoir and allow gradual penetration of a drug into the viable skin layers over a prolonged period of time.

In a topical application, a sufficient amount of the miR-335, an agent capable of up-regulating the expression or activity of miR-335, a histone deacetylase (HDAC) inhibitor or belinostat must penetrate a patient's skin in order to obtain a desired pharmacological effect. It is generally understood that the absorption of drug into the skin is a function of the nature of the drug, the behaviour of the vehicle, and the skin. Three major variables account for differences in the rate of absorption or flux of different topical drugs or the same drug in different vehicles; the concentration of drug in the vehicle, the partition coefficient of drug between the stratum corneum and the vehicle and the diffusion coefficient of drug in the stratum corneum. To be effective for treatment, a drug must cross the stratum corneum which is responsible for the barrier function of the skin. In general, a topical formulation which exerts a high *in vitro* skin penetration is effective *in vivo.* Ostrenga et al (J. Pharm. Sci., 60:1175-1179 (1971) demonstrated that *in vivo* efficacy of topically applied steroids was proportional to the steroid penetration rate into dermatomed human skin *in vitro.*

A skin penetration enhancer which is dermatologically acceptable and compatible with the miR-335, an agent capable of up-regulating the expression or activity of miR-335, a histone deacetylase (HDAC) inhibitor or belinostat can be incorporated into the formulation to increase the penetration of the active compound(s) from the skin surface into epidermal keratinocytes. A skin enhancer which increases the absorption of the active compound(s) into the skin reduces the amount of the miR-335, an agent capable of up-regulating the expression or activity of miR-335, a histone deacetylase (HDAC) inhibitor or belinostat needed for an effective treatment and provides for a longer lasting effect of the formulation. Skin penetration enhancers are well known in the art. For example, dimethyl sulfoxide (U.S. Pat. No. 3,711,602); oleic acid, 1,2-butanediol surfactant (Cooper, J. Pharm. Sci., 73:1153-1156 (1984)); a combination of ethanol and oleic acid or oleyl alcohol (EP 267,617), 2-ethyl-1,3-hexanediol (WO 87/03490); decyl methyl sulphoxide and Azone.RTM. (Hadgraft, Eur. J. Drug. Metab. Pharmacokinet, 21:165-173 (1996)); alcohols, sulphoxides, fatty acids, esters, Azone.RTM., pyrrolidones, urea and polyoles (Kalbitz et al, Pharmazie, 51:619-637 (1996));

Terpenes such as 1,8-cineole, menthone, limonene and nerolidol (Yamane, J. Pharmacy & Pharmocology, 47:978-989 (1995)); Azone.RTM. and Transcutol (Harrison et al, Pharmaceutical Res. 13:542-546 (1996)); and oleic acid, polyethylene glycol and propylene glycol (Singh et al, Pharmazie, 51:741-744 (1996)) are known to improve skin penetration of an active ingredient.

Levels of penetration of the miR-335, an agent capable of up-regulating the expression or activity of miR-335, a histone deacetylase (HDAC) inhibitor or belinostat composition can be determined by techniques known to those of skill in the art. For example, radiolabelling of the active compound, followed by measurement of the amount of radiolabelled compound absorbed by the skin enables one of skill in the art to determine levels of the composition absorbed using any of several methods of determining skin penetration of the test compound. Publications relating to skin penetration studies include Reinfenrath, W G and G S Hawkins. The Weanling Yorkshire Pig as an Animal Model for Measuring Percutaneous Penetration. In: Swine in Biomedical Research (M. E. Tumbleson, Ed.) Plenum, New York, 1986, and Hawkins, G. S. Methodology for the Execution of In Vitro Skin Penetration Determinations. In: Methods for Skin Absorption, B W Kemppainen and W G Reifenrath, Eds., CRC Press, Boca Raton, 1990, pp.67-80; and W. G. Reifenrath, Cosmetics & Toiletries, 110:3-9 (1995).

For some applications, it is preferable to administer a long acting form of the miR-335, an agent capable of up-regulating the expression or activity of miR-335, a histone deacetylase (HDAC) inhibitor or belinostat composition using formulations known in the arts, such as polymers. The miR-335, an agent capable of up-regulating the expression or activity of miR-335, a histone deacetylase (HDAC) inhibitor or belinostat can be incorporated into a dermal patch (Junginger, H. E., in Acta Pharmaceutica Nordica 4:117 (1992); Thacharodi et al, in Biomaterials 16:145-148 (1995); Niedner R., in Hautarzt 39:761-766 (1988)) or a bandage according to methods known in the arts, to increase the efficiency of delivery of the drug to the areas to be treated.

Optionally, the topical formulations described here can have additional excipients for example; preservatives such as methylparaben, benzyl alcohol, sorbic acid or quaternary ammonium compound; stabilizers such as EDTA, antioxidants such as butylated hydroxytoluene or butylated hydroxanisole, and buffers such as citrate and phosphate.

Other therapeutic agents suitable for use herein are any compatible drugs that are effective for the intended purpose, or drugs that are complementary to the retinol formulation. As an example, the treatment with a formulation as set out in this document can be combined with other treatments such as a topical treatment with corticosteroids, calcipotrine, coal tar preparations, a systemic treatment with methotrexate, retinoids, cyclosporin A and photochemotherapy. The combined treatment is especially important for treatment of an acute or a severe skin disease. The formulation utilized in a combination therapy may be administered simultaneously, or sequentially with other treatment, such that a combined effect is achieved.

Modified-release dosage formulations of miR-335, a histone deacetylase (HDAC) inhibitor or belinostat may also be employed. This is in contrast to immediate-release dosage formulations, which release a drug immediately or shortly after its administration. In modified-release dosage formulations, the miR-335, a histone deacetylase (HDAC) inhibitor or belinostat is delivered with a delay after the administration of the miR-335, a histone deacetylase (HDAC) inhibitor or belinostat, or over a prolonged period of time.

Modified-release dosage formulations include extended-release (ER, XR or XL) formulations. They also include sustained-release (SR) formulations, which release the miR-335, a histone deacetylase (HDAC) inhibitor or belinostat at a predetermined rate so as to maintain a drug concentration over a period of time.

Other formulations may include controlled delivery (CD), controlled release (CR), delayed release (DR), extended release (ER), immediate release (IR), long-acting (LA), long-acting release (LAR), modified release (MR), prolonged release (PR), sustained action(SA), sustained release (SR), timed release (TR), extended release (XL), extended release (XR) and extended release (XT) formulations.

Such formulations are well known in the art and are described for example in Remington: The Science and Practice of Pharmacy, Nineteenth Edn, 1995, Mack Publishing Co, Pennsylvania, USA.

### ADMINISTRATION

The miR-335, a histone deacetylase (HDAC) inhibitor or belinostat composition may be applied to skin using any suitable treatment regime.

The composition may be given in a single dose or multiple doses. The single dose may be administered daily, or multiple times a day, or multiple times a week, or monthly or multiple times a month. The composition may be given in a series of doses. The series of doses may be administered daily, or multiple times a day, weekly, or multiple times a week, or monthly, or multiple times a month. Thus, one of skill in the art realizes that depending upon the skin type, location, health of the subject, etc., the composition described here may be administered for any given period of time until the treatment, prevention or alleviation of the dermatological condition is achieved at least by 5%, 10%, 20%, 30%, 40%, 50%, 55%, 60%, 65%, 70%, 75%, 80%, 85%, 90%, 95% or 100% or any range in between.

The miR-335, a histone deacetylase (HDAC) inhibitor or belinostat composition may be applied at least once a week, such as at least every two days, or at least once each day. For example, application may be twice per day.

In general, treatment using the miR-335, a histone deacetylase (HDAC) inhibitor or belinostat composition described here may be continued indefinitely. Alternatively, the treatment may be repeated only for a limited period, e.g. several weeks or months. Treatment may then be repeated for a similar period at a later date.

After application to the skin, the composition may be rinsed off, or may be left on the skin. If the composition is to be rinsed off after application, the composition may be left on for a minimum period of time before rinsing. An example period of time is more than 30 seconds, such as more than 1 minute, such as more than 3 minutes.

The product may be massaged into the skin, most commonly into the scalp, during application, such as for at least 5 seconds, such as for at least 20 seconds.

### KITS

We further describe a kit which is not part of the invention comprising (a) miR-335, a histone deacetylase (HDAC) inhibitor or belinostat (or a salt, hydrate, or solvate thereof), or a composition comprising miR-335, a histone deacetylase (HDAC) inhibitor or belinostat (or a salt, hydrate, or solvate thereof), e.g., preferably provided in a suitable container and/or with suitable packaging; and (b) instructions for use, e.g., written instructions on how to administer the compound or composition in accordance with the methods described here for the purposes described.

The written instructions may also include a list of indications for which the active ingredient is a suitable treatment.

### DETECTION AND DIAGNOSTIC METHODS

### Detection of Expression of miR-335

We show in the Examples that the expression of miR-335 in atopic dermatitis is downregulated when compared to normal skin.

We demonstrate that, in healthy skin, proliferating cells in the basal layer of the epidermis express low levels of miR-335, while high levels of miR-335 are expressed in suprabasal layers. We demonstrate that, in AD lesional skin, miR-335 expression is lost in all layers, including both basal and suprabasal layers.

Accordingly, we provide for but do not claim a method of diagnosis of a dermatological condition such as atopic dermatitis in a cell or tissue of an individual.

Detection of miR-335 expression, activity or amount may be used to provide a method of determining the lesional state of a cell. The cell may comprise a suprabasal cell of the epidermis. Thus, a lesional cell is a suprabasal cell with low levels of miR-335 expression, activity or amount compared to a normal suprabasal cell of the epidermis.

Such detection of miR-335 expression in for example a suprabasal cell may also be used to determine whether a cell will become lesional. Thus, detection of a low level of miR-335 expression, amount or activity of miR-335 in the cell may indicate that the cell is likely to be or become lesional. Similarly, if a cell has a normal or high level of miR-335 expression, amount or activity, the cell is not or is not likely to be lesional.

Detection of miR-335 expression, amount or level in for example a suprabasal cell may be used to determine the likelihood of success of a particular therapy in an individual with a dermatological condition such as atopic dermatitis.

The diagnostic methods described in this document may be combined with the therapeutic methods described. Thus, we provide an agent for use, as defined herein, in a method of treatment, prophylaxis or alleviation of a dermatological condition such as atopic dermatitis in an individual, the method comprising detecting modulation of expression, amount or activity of miR-335 in a cell of the individual and administering an appropriate therapy to the individual based on the level of expression, amount or activity.

The presence and quantity of miR-335 nucleic acids may be detected in a sample as described in further detail below. Thus, the miR-335 associated diseases, including a dermatological condition such as atopic dermatitis, can be diagnosed by methods comprising determining from a sample derived from a subject an abnormally decreased or increased expression, amount or activity, such as a decreased expression, amount or activity, of the miR-335 nucleic acid.

The sample may comprise a cell or tissue sample from an organism or individual suffering or suspected to be suffering from a disease associated with decreased, reduced or otherwise abnormal miR-335 expression, amount or activity, including spatial or temporal changes in level or pattern of expression, amount or activity. The sample may comprise a basal cell of the epidermis or a suprabasal cell of the epidermis. The level or pattern of expression, amount or activity of miR-335 in an organism suffering from or suspected to be suffering from such a disease may be usefully compared with the level or pattern of expression, amount or activity in a normal organism as a means of diagnosis of disease.

The sample may comprise a cell or tissue sample from an individual suffering or suspected to be suffering from a dermatological condition such as atopic dermatitis, such as a skin tissue or cell sample. The cell or tissue may comprise a basal cell of the epidermis or a suprabasal cell of the epidermis.

In some embodiments, an decreased level of expression, amount or activity of miR-335 is detected in the sample. The level of miR-335 may be decreased to a significant extent when compared to normal cells, or cells from individuals known not to be suffering from a dermatological condition such as atopic dermatitis. Such cells may be obtained from the individual being tested, or another individual, such as those matched to the tested individual by age, weight, lifestyle, etc.

In some embodiments, the level of expression, amount or activity of miR-335 is decreased by 10%, 20%, 30% or 40% or more. In some embodiments, the level of expression, amount or activity of miR-335 is decreased by 45% or more, such as 50% or more.

The expression, amount or activity of miR-335 may be detected in a number of ways, as known in the art, and as described in further detail below. Typically, the amount of miR-335 in a sample of tissue from an individual is measured, and compared with a sample from an unaffected individual.

Detection of the amount, activity or expression of miR-335 may be used to grade a dermatological condition such as atopic dermatitis. For example, a low level of amount, activity or expression of miR-335 may indicate a more severe case of a dermatological condition such as atopic dermatitis. Similarly, a high level of amount, activity or expression of miR-335 may indicate a milder or less severe case of a dermatological condition such as atopic dermatitis. Such a grading system may be used in conjunction with established grading systems for a dermatological condition such as atopic dermatitis.

Levels of miR-335 expression may be determined using a number of different techniques.

### Measuring Expression of miR-335 at the RNA level

miR-335 gene expression can be detected at the RNA level.

We disclose but do not claim a method of detecting the presence of a miR-335 nucleic acid in a sample. by contacting the sample with at least one nucleic acid probe which is specific for the miR-335 and monitoring said sample for the presence of the miR-335. For example, the nucleic acid probe may specifically bind to the miR-335, or a portion of it, and binding between the two detected; the presence of the complex itself may also be detected.

Thus, the amount of miR-335 may be measured in a sample. miR-335 may be assayed by in situ hybridization, Northern blotting and reverse transcriptase--polymerase chain reaction. Nucleic acid sequences may be identified by in situ hybridization, Southern blotting, single strand conformational polymorphism, PCR amplification and DNA-chip analysis using specific primers. (Kawasaki, 1990; Sambrook, 1992; Lichter et al, 1990; Orita et al, 1989; Fodor et al., 1993; Pease et al., 1994).

miR-335 RNA may be extracted from cells using RNA extraction techniques including, for example, using acid phenol/guanidine isothiocyanate extraction (RNAzol B; Biogenesis), or RNeasy RNA preparation kits (Qiagen).Typical assay formats utilising ribonucleic acid hybridisation include nuclear run-on assays, *in situ* hybridisation, RT-PCR and RNase protection assays (Melton et al., Nuc. Acids Res. 12:7035. Methods for detection which can be employed include radioactive labels, enzyme labels, chemiluminescent labels, fluorescent labels and other suitable labels.

Each of these methods allows quantitative determinations to be made, and are well known in the art. An example of a detection method suitable for the compositions and methods described in this document is *in situ* hybridisation. Methods and detailed protocols for *in situ* hybridisation for detecting microRNA are known in the art and are described for example in Nielsen (2012) MicroRNA In Situ Hybridization, pages 67-84 in Jian-Bing Fan (ed.), Next-Generation MicroRNA Expression Profiling Technology: Methods and Protocols, Methods in Molecular Biology, vol. 822 and Urbanek et al (2015) Small RNA Detection by in Situ Hybridization Methods, Int. J. Mol. Sci. 16, 13259-13286.

Decreased or increased miR-335 expression, amount or activity can therefore be measured at the RNA level using any of the methods well known in the art for the quantitation of polynucleotides. Any suitable probe from a miR-335 sequence, for example, any portion of a suitable human miR-335 sequence may be used as a probe.

Typically, RT-PCR is used to amplify RNA targets. In this process, the reverse transcriptase enzyme is used to convert RNA to complementary DNA (cDNA) which can then be amplified to facilitate detection.

Many DNA amplification methods are known, most of which rely on an enzymatic chain reaction (such as a polymerase chain reaction, a ligase chain reaction, or a self-sustained sequence replication) or from the replication of all or part of the vector into which it has been cloned.

Many target and signal amplification methods have been described in the literature, for example, general reviews of these methods in Landegren, U. et al., Science 242:229-237 (1988) and Lewis, R., Genetic Engineering News 10:1, 54-55 (1990).

For example, the polymerase chain reaction may be employed to detect miR-335.

The "polymerase chain reaction" or "PCR" is a nucleic acid amplification method described *inter alia* in U.S. Pat. Nos. 4,683,195 and 4,683,202. PCR can be used to amplify any known nucleic acid in a diagnostic context (Mok et al., 1994, Gynaecologic Oncology 52:247-252). Self-sustained sequence replication (3SR) is a variation of TAS, which involves the isothermal amplification of a nucleic acid template via sequential rounds of reverse transcriptase (RT), polymerase and nuclease activities that are mediated by an enzyme cocktail and appropriate oligonucleotide primers (Guatelli et al., 1990, Proc. Natl. Acad. Sci. USA 87:1874). Ligation amplification reaction or ligation amplification system uses DNA ligase and four oligonucleotides, two per target strand. This technique is described by Wu, D. Y. and Wallace, R. B., 1989, Genomics 4:560. In the Qβ Replicase technique, RNA replicase for the bacteriophage Qβ, which replicates single-stranded RNA, is used to amplify the target DNA, as described by Lizardi et al., 1988, Bio/Technology 6:1197.

A PCR procedure basically involves: (1) treating extracted DNA to form single-stranded complementary strands; (2) adding a pair of oligonucleotide primers, wherein one primer of the pair is substantially complementary to part of the sequence in the sense strand and the other primer of each pair is substantially complementary to a different part of the same sequence in the complementary antisense strand; (3) annealing the paired primers to the complementary sequence; (4) simultaneously extending the annealed primers from a 3' terminus of each primer to synthesize an extension product complementary to the strands annealed to each primer wherein said extension products after separation from the complement serve as templates for the synthesis of an extension product for the other primer of each pair; (5) separating said extension products from said templates to produce single-stranded molecules; and (6) amplifying said single-stranded molecules by repeating at least once said annealing, extending and separating steps.

Reverse transcription-polymerase chain reaction (RT-PCR) may be employed. Quantitative RT-PCR may also be used. Such PCR techniques are well known in the art, and may employ any suitable primer from a miR-335 sequence.

Alternative amplification technology can also be exploited. For example, rolling circle amplification (Lizardi et al., 1998, Nat Genet 19:225) is an amplification technology available commercially (RCAT^{™}) which is driven by DNA polymerase and can replicate circular oligonucleotide probes with either linear or geometric kinetics under isothermal conditions. A further technique, strand displacement amplification (SDA; Walker et al., 1992, Proc. Natl. Acad. Sci. USA 80:392) begins with a specifically defined sequence unique to a specific target.

### Detecting Expression of miR-335-Induced Polypeptides

As shown in the Examples, miR-335 inhibits the expression of SOX6.

Accordingly, miR-335 expression may be detected using the expression of SOX6 as proxy, at either the nucleic acid level or the polypeptide level.

### DIAGNOSTIC KITS

We also disclose but do not claim diagnostic kits for detecting a dermatological condition such as atopic dermatitis in an individual, or susceptibility to a dermatological condition such as atopic dermatitis in an individual.

The diagnostic kit may comprise means for detecting expression, amount or activity of miR-335 in the individual, by any means as described in this document. The diagnostic kit may therefore comprise any one or more of the following: a miR-335 or a fragment thereof; a complementary nucleotide sequence to miR-335 nucleic acid or a fragment thereof.

The diagnostic kit may comprise instructions for use, or other indicia. The diagnostic kit may further comprise means for treatment or prophylaxis of a dermatological condition such as atopic dermatitis, such as any of the compositions described in this document, or any means known in the art for treating a dermatological condition such as atopic dermatitis. The diagnostic kit may comprise a therapeutic composition comprising miR-335, a histone deacetylase (HDAC) inhibitor or belinostat.

### EXAMPLES

### Example 0. Introduction

Here, we investigate microRNAs which are critical to maintain skin barrier function, and identify miR-335 as a key driver of keratinocyte differentiation and cornification.

*In silico* predictions, followed by experimental validation, establish transcription factor SOX6 as a direct target for miR-335 repression. This regulatory relationship, which promotes epidermal differentiation, is disrupted in AD.

In healthy epidermis, miR-335 is abundantly expressed and SOX6 is absent; contrary to this, in patient lesional skin sections, loss of miR-335 is observed in tandem with aberrant SOX6 upregulation. SOX6 suppresses epidermal differentiation by recruiting SMARCA complex components, which epigenetically silence critical genes involved in keratinocyte differentiation. The resultant skin barrier defect can be therapeutically reversed by restoring miR-335 expression. miR-335 is epigenetically regulated by histone deacetylases (HDACs), and a screen for suitable HDAC inhibitors identified belinostat as a candidate drug which can restore epidermal miR-335 expression. This is of clinical significance not only as an treatment for AD, but also as a potential means of stopping the atopic march and further progression of this systemic allergic disease.

We identified a microRNA, miR-335, as a key driver of the keratinocyte differentiation and cornification, which is required for establishment of a healthy skin barrier.

However in AD lesional skin expression of miR-335 is significantly downregulated. In the absence of miR-335, we observed sustained expression of its downstream targets, SOX6 and CASP7.

Delineating the mechanism, we demonstrate how SOX6-mediated recruitment of SMARCA complex leads to epigenetic silencing of genes critical for epithelial differentiation resulting in a barrier defect.

Furthermore, in the absence of miR-335, persistent expression of pro-inflammatory CASP7, facilitates increased expression of inflammatory cytokines crafting an inflammatory microenvironment.

Thus, miR-335 drives keratinocyte differentiation, and concomitantly plays a role in the resolution of inflammation.

Understanding how these are dysregulated in atopic dermatitis, our objective was to restore miR-335, repair barrier defects and keep inflammation at bay.

Our data also demonstrates that miR-335 biogenesis is epigenetically regulated via histone deacetylases (HDAC).

### Example 1. Materials and Methods: Cell Culture and Transfection

N/TERT-1 keratinocytes (a gift from James Rheinwald) were grown in keratinocyte serum-free medium (KSFM) (Life Technologies) supplemented with 0.2 ng/mL epidermal growth factor (EGF), 25 µg/mL bovine pituitary extract (BPE), 0.4 mM calcium chloride (CaCl2) and 1% penicillin/streptomycin. When high density cultures were required for experiments, N/TERT-1 cells were grown in DF-K medium of glutamine-free Dulbecco's modified Eagle's medium (DMEM; Gibco) and KSFM supplemented with 0.2 ng/ml EGF, 25 µg/ml BPE, 2 mM L-glutamine, 0.15 mM CaCl2 and 1% penicillin/streptomycin). HEK293T cells (Clontech) were cultured in DMEM supplemented with 10% fetal bovine serum (Gibco), 4 mM L-glutamine and 1 mM sodium pyruvate. Cultures were maintained at 37°C in a humidified incubator containing 5% CO2.

N/TERT-1 keratinocytes were grown in six-well plates to 30-40% confluency before transfection. For miR-335 overexpression studies, N/TERT-1 cells were transfected with miR-335 mimic or negative control mimic (Dharmacon), at a final concentration of 30 nM using Lipofectamine^{®} RNAiMAX reagent (Life Technologies) according to manufacturer's protocol. For knockdown studies, short interfering RNAs (siRNAs) against *SOX6, CASP7, MEST, HELLS, SMARCA4, PNN* or *SMARCC1* (Dharmacon) were transfected into N/TERT-1 in the same manner as described for miRNA transfection. A nontargeting siRNA was transfected as negative control. Total RNA and/or protein were harvested at 48 hours post transfection and subjected to quantitative real time PCR and/or western blotting.

### Example 2. Materials and Methods: Preparation of Lentiviral Stocks and Transduction

Third-generation lentiviral particles were produced in HEK293T cells (Clontech) using calcium phosphate transfection, with co-transfection of lentiviral vectors and packaging mix made of three constructs, namely pMDLg/pRRE (#12251, Addgene), pRSV-Rev (#12253, Addgene) and pMD2.G (#12259, Addgene). Control viruses were prepared using empty vector constructs. For pTRIPZ plasmids, the co-transfection was done with Trans-lentiviral packaging mix (Dharmacon) using Lipofectamine 2000 (Thermo Fisher Scientific) into HEK293T cells according to manufacturer's instructions. Viral particle-containing supernatants were harvested 48 hours and 72 hours after transfection. Viral supernatants were filtered through 0.22 µM membrane to remove non-adherent cells and debris, and subsequently concentrated via ultracentrifugation at 19600 rpm for 4 hours at 4°C. Viral particles were then resuspended in Hank's Balanced Salt Solution (Sigma-Aldrich). To determine the titre of each virus, viral titration was performed in HEK293T cells after transduction with serial dilutions of the lentiviral stocks according to the protocol described by Tiscornia *et al* (2006). The viral titre was calculated based on the percentage of GFP-positive cells as revealed by flow cytometry analysis. Lentiviral transduction was performed by incubating N/TERT-1 cells with 10 µg/mL polybrene and purified lentiviral particles at an infection ratio of 4 transduction units (TUs) per cell. Stably transduced cells were selected either using GFP sorting (for pCDH constructs) or using puromycin treatment for 5 days (for control constructs).

### Example 3. Materials and Methods: miRNA Profiling

Total RNA was extracted from skin biopsies using TRIzol^{®} reagent followed by Exiqon miRCURY RNA column purification. Total RNA was labelled using miRCURY LNA^{™} microRNA Hi-Power Labelling Kit (Exiqon) according to manufacturer's protocol. The labelled samples were hybridized on the miRCURY LNA^{™} microRNA Array (6th generation) with probes against 1223 known human mature miRNAs. Raw intensities from all samples were background subtracted, normalized using the global locally weighted scatterplot smoothing (lowess) regression method and log2 transformed. Differential miRNA expression analyses were performed using Partek Genomics Suite software.

### Example 4. Materials and Methods: Microarray Analysis

Total RNA was converted to biotinylated cRNA using TargetAmp Nano-g Biotin-aRNA labeling kit (Epicenter). cRNA was purified using RNeasy Mini Kit (Qiagen). cRNA was hybridized on HumanHT-12 V4 Expression BeadChip Kit (Illumina) according to the manufacturer's instructions. The raw data was extracted, background subtracted and normalized using Illumina BeadStudio. Differential gene expression analysis was performed using Partek Genomics Suite software.

### Example 5. Materials and Methods: miRNA in situ Hybridization

Paraffin wax-embedded skin tissue sections (5 µm) were dewaxed, rehydrated and boiled in pre-treatment solution (Panomics) for 5 minutes and subsequently treated with protease (Panomics) for 30 minutes at 37°C. Locked nucleic acid (LNA) probes were then added to the sections and incubated at 51°C for 4 hours. The LNA probes used are specific to miR-335 and scrambled (non-targeting) sequences and are 5' and 3' digoxigenin- (DIG-) labelled. After washing the sections sequentially with 5X saline-sodium citrate (SSC), 1X SSC and 0.3X SSC buffer, they were blocked with 10% goat serum and incubated with anti-DIG alkaline phosphatase (Roche) overnight at 4°C. Fast Red Substrate (Panomics) were used to detect miRNA-bound LNA probes. Tissue sections were counterstained with 4',6-diamidino-2-phenylindole dihydrochloride (DAPI) (Sigma-Aldrich) and mounted with FluorSaveTM reagent (EMD Millipore). The slides were examined with an FV1000 inverted confocal microscope (Olympus) and images were acquired using Olympus FluoView with TRITC and DAPI filters.

### Example 6. Materials and Methods: Immunohistochemistry

Paraffin wax-embedded skin tissue sections (5 µm) were dewaxed and rehydrated through decreasing concentration of ethanol. Endogenous peroxidases in tissue sections were quenched by 3% H202 in absolute methanol for 30 minutes. The sections were then boiled in antigen retrieval solution (pH 6, DAKO). Sections were then blocked in 10% goat serum for 30 minutes and incubated with primary antibodies overnight at 4°C. After incubation with primary antibodies, tissues were incubated with anti-rabbit or -mouse Envision-labelled polymer reagents (DAKO) for 1 hour at room temperature. The staining was visualized by 3,3'-diaminodbenzidine (DAB) substrate chromogen kit (DAKO). Slides were counterstained with haematoxylin and examined with a Zeiss AxioImager Z1 upright light microscope after mounting with CytosealTM60 mounting medium (Richard Allan Scientific). Images were acquired using Zeiss Zen software.

### Example 7. Materials and Methods: Immunocytochemistry

Cells were fixed and permeabilized with ice-cold acetone/methanol for 10 minutes. After washing in phosphate buffered saline (PBS), the cells were incubated with 10% goat serum for 30 minutes. The cells were subsequently incubated with primary antibodies for 2 hours at room temperature or overnight at 4°C. After washing three times with PBS with 0.1% Tween 20, the cells were incubated in dark with Alexa Fluor secondary antibodies (Invitrogen) for an hour. Nuclei were stained using DAPI. After washing with PBS, the cells were mounted onto slides with FluorSave reagent (EMD Millipore). Images were taken with an FV1000 inverted confocal microscope (Olympus) using Olympus FluoView.

### Example 8. Materials and Methods: Quantitative Real-Time PCR

Total RNA was isolated using miRCURY^{™} RNA isolation kit (Exiqon). For miRNA quantification, cDNA was synthesized using Taqman^{®} miRNA reverse transcription kit (Life Technologies) and the expression level of miRNAs was quantified by TaqMan Gene Expression Assays (Applied Biosystems) in 7900 fast RT-PCR system (Applied Biosystems) using Taqman miRNA-specific primers. For mRNA expression, cDNA was synthesized using SuperScript^{®} III Reverse Transcriptase (Life Technologies) according to manufacturer's protocol. The expression levels of mRNAs were measured using SYBR green PCR Master Mix (Applied Biosystems). U6 and RPLP0 were used as endogenous normalization controls for miRNA and protein-coding genes, respectively. The expression of a gene/miRNA was defined from threshold cycle (Ct), and relative transcript abundance was calculated using ddCT method. All reactions were run in triplicates. The primers were designed using the Primer3 software and the NCBI design primer tools. The primers were checked for their GC content and any secondary structure formation using OligoCalc.

### Example 9. Materials and Methods: Western Blotting

Whole cell lysates were extracted using RIPA buffer (50 mM Tris-HCl pH 7.4, 300 mM NaCl, 1% Nonidet P-40 (NP40), 1% sodium deoxycholate, 0.05% sodium dodecyl sulphate (SDS) and 10% glycerol) supplemented with protease inhibitor (Calbiochem). The concentration of the protein samples was measured by Bradford protein assay (Bio-Rad). 20-30µg protein samples were resuspended with 2x Laemmli buffer, separated on a 4-15% TGXTM precast protein gel (Biorad) by SDS-PAGE and electrophoretically transferred to PVDF membranes (Millipore). After incubation with 5% skimmed milk in Tris-buffered saline with 1% Tween (TBST) for 30 minutes, membranes were incubated with primary antibodies at 4°C overnight. Membranes were washed three times for 15 minutes and incubated with HRP-conjugated anti-rabbit or anti-mouse antibodies. Blots were washed three times with TBST and developed through autoradiography using ECL western detection reagent (Millipore Crescendo). Beta-actin was used as a protein loading control.

### Example 10. Materials and Methods: Dual Luciferase Reporter Assay

Wild-type 3' UTR report constructs of SOX6 and CASP7 were co-transfected with pCDH or pCDH-335 plasmids into HEK293T cells using Lipofectamine 2000 (Thermo Scientific). The firefly and *Renilla* luminescence were detected 24 hours after transfection using Dual-Luciferase reporter assay (Promega).

### Example 11. Materials and Methods: Chromatin Immunoprecipitation

Cells were crosslinked with 1.5% formaldehyde for 10 minutes at 37°C. The reaction was stopped by adding glycine to the final concentration of 125 mM for 5 minutes at 37°C. Fixed cells were washed twice with ice-cold PBS, scraped and centrifuged. The pellets were then incubated with Buffer A (10 mM HEPES Ph7.9, 10 mM KCl, 0.1 mM EGTA, 1 mM DTT and 0.5 mM PMSF) supplemented with 1X protease inhibitor cocktail (Roche) and 10% glycerol on ice for 30 min, followed by 0.5% NP-40 for 5 minutes to lyse cytoplasmic membrane. The cells were then centrifuged at 1500×g for 5 minutes to pellet down nuclei. Nuclear pellet was then lysed in ChIP buffer (50 mM Tris pH 7.4, 150 mM NaCl, 5 mM EDTA, 0.5% NP-40, 1% Triton X-100, 0.05% SDS, 1× protease inhibitor cocktail). Nuclear chromatin was sheared by sonication for 15 min (30 sec on and 30 sec off) in Bioruptor^{®} water bath sonicator. The sonicated samples were centrifuged, and the supernatant containing the sheared chromatin was harvested. For each ChIP reaction, 250 µl of chromatin extract was incubated with appropriate antibodies at 4°C overnight on a rotary wheel. 50 µl of Protein A sepharose resin (precoated with 0.5% BSA and 0.2 mg/mL tRNA) was then added to the immunoprecipitated samples and incubated for 2 hours. The samples were then washed 5 times with ChIP buffer and the final wash was done with PBS. 100 µl of 10% Chelex slurry was added to each reaction and boiled for 5 minutes. The supernatant was collected, and the second elution was done with 100 µl nuclease-free water. The sample was then treated with RNase A (Roche) at 37°C for 2 hours followed by Proteinase K (Roche) at 55°C overnight. The eluted samples were purified using the QIAquick PCR purification kit (Qiagen) according to the manufacturer's protocol. For total chromatin input, 25 µl of chromatin extract was washed with ice-cold 75% ethanol and spin at 16000xg for 10 minutes at 4°C. 100ul 10% Chelex slurry was added to each sample and boiled for 5 minutes. The eluted samples were then subjected to RNase A and Proteinase K treatment as described above. The resulting DNA was diluted 5 times and 4 µl of each sample was used for real-time PCR. Fold enrichment of the genes was calculated using ddCt method with input Ct values as normalization control.

### Example 12. Materials and Methods: Co-immunoprecipitation

Dynabeads Protein A (Invitrogen) were prepared according to the manufacturer's protocol. The nuclear lysates were incubated with DynaBeads Protein A coated with appropriate antibodies for 4 hours at 4°C. After washing the beads for 5 times in wash buffer (PBST) to remove non-specific proteins, the beads-bound proteins were analysed by mass spectrometry. For the validation of co-immunoprecipitated proteins by western blotting, proteins were eluted in 2x Laemli buffer at 95°C for 5 minutes. The eluted protein were subjected to western blot as per standard protocols.

### Example 13. Materials and Methods: Mass Spectrometry

Proteins bound to DynaBeads were digested on-bead according to Duan *et al.* (2009). The digested peptides were analyzed on Orbitrap Fusion Tribrid mass spectrometer coupled to a proxeon EASY-nLC 1000 liquid chromatography. Proteome Discoverer was used to analyze the raw data. The candidates presenting unique peptides > 2 and peptide spectrum matches (PSM) > 15 were selected and subjected to co-immunoprecipitation validation.

### Example 14. Materials and Methods: Cornified Envelope Assay

N/TERT-1 keratinocytes were grown in DF-K media until confluent, and then shifted to high Ca²⁺ conditions (1.5 mM) for a further 7 days in fresh DF-K media to induce terminal differentiation and cornified envelope formation, and the number of cornified envelopes (CEs) assessed following the method of Rice et al. In brief, cells were trypsinized and resuspended at 2.0 x 10⁶ cells/ml in dissociation buffer containing 0.1 M Tris-HCl buffer pH 8.0, 2% SDS and 20 mM dithiothreitol. CEs were harvested by boiling the samples for 15 minutes at 100°C. Detergent- and reducing agent-resistant CEs were collected as an insoluble pellet by centrifugation at 4000×g for 10 minutes. CEs were counted with a hemocytometer, and CE formation was expressed as a percentage of input cell number.

### Example 15. Materials and Methods: HDAC Inhibitor Screening

A panel of 42 HDAC inhibitors (HDACi) covering the majority of type I, II and pan HDACis were purchased from Selleckchem. N/TERT-1 grown to confluence were treated with HDACi at a final concentration of 1µM from a 100µM stock. Control wells were treated with DMSO at a final concentration of 1%. After 48hrs of treatment, cells were either processed for total RNA isolation or fixed and stained for human involucrin by immunocytochemistry.

### Example 16. Materials and Methods: Ex-vivo Human Skin Organ Culture Model

Human skin organ culture was performed following Moll et al. [27]. Briefly, clinically discarded abdominal skin was collected within 3hrs post-surgery. Excess fat was trimmed using scissors and 8mm circular explants were cut out using a commercial biopsy punch. Explants were placed on 6 well membrane inserts (4uM PET membrane) and cultured at air-liquid interface. Belinostat and mocetinostat were dissolved in acetone at a final concentration of 1mM and 10ul of this mix was applied directly on the epidermal side of the biopsy and allowed to dry. Treatment with 10ul of acetone alone was used as mock treatment. Organ cultures were treated daily for a period of six days. After this period, skin biopsies were either processed for total RNA isolation as described earlier [52] or were fixed in 10% neutral buffered formalin and processed into FFPE sections.

### Example 17. Materials and Methods: Bioinformatics Analysis Software

DAVID (https://david.ncifcrf.gov/) was used to perform gene ontology. TargetScan (http://www.targetscan.org/), miRANDA (http://www.microrna.org) and PITA (https://genie.weizmann.ac.il/pubs/mir07/mir07_data.html) were used for miRNA target gene prediction. ProteINSIDE (www.proteinside.org) was used to evaluate the enrichment of nuclear proteins in the mass spectrometry data set.

### Example 18. Materials and Methods: Statistical Analysis

All quantitative data were presented as mean ± standard error. Statistical analysis was performed with two-tailed Student's t test when comparing two samples. Values of p< 0.05 were considered statistically significant.

### Example 19. Results: A Screen of miRNAs in AD Identifies miR-335 as an Epithelial Differentiation Factor

MicroRNA microarray analysis comparing AD lesional versus normal healthy skin revealed multiple differentially-expressed miRNAs (Figure 7A).

The microarray data was cross-checked using quantitative real-time PCR (qRT-PCR) on RNA isolated from 10 lesional skin samples and 7 healthy controls. We found miR-335 to be the most consistent differentially-expressed miRNA in these samples - miR-335 is significantly downregulated in AD lesions relative to healthy skin (Student's t-test, *P* < *0.01;* Figure 1A).

To characterize the expression pattern of miR-335 we performed *in situ* hybridization on sections from AD lesional skin and healthy skin using locked nuclei acid (LNA) probes highly specific for mature miR-335. Significant expression of miR-335 was apparent in the epidermis of unaffected individuals, whilst in contrast very little or no miR-335 was detectable in AD lesional sections (Figure 1B).

Intriguingly, high expression of miR-335 was limited to the suprabasal (differentiating) layers of the epidermis, whereas the miR-335 signal was much lower in the basal (undifferentiated) layer of the epidermis in normal healthy skin (Figure 1B). Expression of miR-335 only in differentiation -committed epidermal cells suggests a role for miR-335 in differentiation and maintenance of epidermal homeostasis.

We evaluated the role of miR-335 in epidermal differentiation by carrying out a microarray analysis on N/TERT-1 cells transfected with miR-335 mimics. N/TERT-1 is an immortalized human keratinocyte cell line [17], which does not express miR-335 in its undifferentiated state (Figure 7B).

Total RNA from N/TERT-1 cells transfected with control or miR-335 mimics (Figure 7C) were subjected to microarray analysis and gene expression profile comparison. Gene set enrichment analysis revealed signatures of human keratinocyte differentiation that were enriched in N/TERT-1 cells transfected with miR-335 mimics. This included a subset of genes that were significantly enriched in GO terms related to keratinization and peptide cross-linking (Figure 1C).

A cohort of genes essential for cornified envelope formation, including *IVL, SPRR1A, SPRR1B, SPRR2E, SPRR2F* and *TGM1,* all genes in the epidermal differentiation complex on chromosome 1q, were significantly upregulated in N/TERT-1 cells expressing miR-335, compared to the control cells (Figure 1D).

Microarray results were further validated by qRT-PCR using biological replicates for this subset of genes (Figure 1E). We performed a cornified envelope assay on N/TERT-1 cells transfected with control or miR-335 mimics to investigate the role of miR-335 in keratinocyte differentiation and cornification.

Cornified envelopes are a well-established feature of terminal keratinocyte differentiation [18]. They are formed by transglutaminase-catalyzed cross-linking of keratinocyte differentiation proteins, which includes Involucrin (IVL), Small Proline Rich Proteins (SPRR) and other proteins.

Phase-contrast microscopy revealed an increase in the number of terminally differentiated cells, seen as cornified envelopes, in cells transfected with miR-335 mimics compared to control cells (Figure 1F and Figure 1G), supporting our hypothesis that miR-335 is crucial for keratinocyte terminal differentiation and barrier formation.

### Example 20. Results: SOX6 is a Direct Target of miR-335

To further evaluate the molecular pathways used by miR-335 to induce keratinocyte differentiation and cornification, we used an integrated genomics, bioinformatics, and experimental approach to identify its targets.

We curated our microarray expression data for genes significantly downregulated by miR-335 and overlapped these hits of miR-335 predicted target genes from available algorithms and shortlisted 30 genes, including the transcription factor *SOX6.*

As depicted in the heatmap, we identified *SOX6* as a bona fide target of miR-335.

SOX6 is significantly downregulated in N/TERTs expressing miR-335 when compared to those transfected with the scrambled control (Figure 2A), and has binding sites for miR-335 in its 3'-UTR (Figure 2B).

To confirm that *SOX6* is a direct target of miR-335, we cloned its 3'-UTR into a luciferase reporter construct. Co-transfection of miR-335 mimics with SOX6 wild-type 3'-UTRs significantly reduced luciferase reporter activity. On the contrary, when the miR-335 binding site was mutated, we did not see a change in the reporter activity, confirming that miR-335 directly binds to the site of interest (Figure 2C).

A clear inverse correlation in the expression pattern of miR-335 and SOX6 was observed in normal skin sections unaffected by AD, with intense nuclear immunohistochemical staining for SOX6 in the basal layer of the epidermis coinciding with little or no miR-335 (Figure 2D, upper panels). Nuclear staining for SOX6 was absent from suprabasal layers in the normal epidermis.

In AD lesional skin, where miR-335 expression is lost, nuclear SOX6 is expressed throughout the epidermis (Figure 2D, lower panels). We also observed that SOX6 transcript and protein abundance in N/TERT-1 cells are reduced in response to miR-335 mimic transfection (Figure 3A and Figure 3B).

### Example 21. Results: miR-335 Induces Transcriptomic Landscapes Characteristic of Epidermal Differentiation by Targeting SOX6

Having established SOX6 as a direct target of miR-335, we set out to evaluate its functional role in the epidermis.

Specific short hairpin RNAs (shRNAs) were used to knock down endogenous SOX6 in the N/TERT-1 cell line (Figure 8A and Figure 8B). ShSOX6 knockdown substantially decreased cell proliferation relative to that observed with a scrambled control (Figure 3C and Figure 3D).

We used genome-wide expression profiling of control and shSOX6 cells to elucidate the molecular mechanism by which SOX6 regulates differentiation. This analysis revealed expression differences in a subset of genes which were significantly enriched in GO terms related to terminal differentiation (epidermal development, differentiation, keratinization and peptide cross-linking) (Figure 3E).

Microarray results were validated by qRT-PCR using biological replicates for a subset of overlapping genes including *IVL, SPRR1A, SPRR1B, SPRR2E, SPRR2F* and *TGM1* (Figure 3F). From these data, we infer that SOX6 suppresses molecular pathways driving epidermal differentiation. SOX6 knockdown in N/TERT-1 cells phenocopies the effect of miR-335 transfection.

The transcriptomic response to SOX6 knockdown was reflected in the morphology of post-confluent shSOX6 keratinocytes, which displayed more stratifying cells after SOX6 silencing, implying higher propensity of these cells to differentiate.

To investigate whether SOX6 knockdown can promote cornification, we measured cornified envelope formation in both shSOX6-and scrambled control-transduced keratinocytes. ShSOX6 cells produced more cornified envelopes relative to controls, indicating that SOX6 suppresses differentiation (Figure 3G). SOX6 downregulation in the epidermis is thus likely to be crucial for skin maturation and healthy barrier formation.

Given these findings that SOX6, a known transcription factor, acts to suppress epidermal differentiation, we looked for evidence that SOX6 directly binds to promoters of differentiation-associated genes. The promoters of such genes were screened for consensus SOX6 binding sequences [19].

We found putative SOX6 binding sites upstream of the transcription start sites in promoters of *IVL* (involucrin), *SPRR2F* (small proline rich protein 2F) and *TGM1* (transglutaminase-1).

Chromatin immunoprecipitation (ChIP) was used to study the occupancy of SOX6 at these sites; N/TERT-1 nuclear extracts were subjected to ChIP using antibodies against SOX6. Quantitative PCR analysis on these samples revealed SOX6 enrichment in the promoters of all three genes (Figure 3H).

We confirmed the specificity of this interaction by showing that SOX6 is not enriched upstream of the control genes *RPLP0* and *KRT14,* which are expressed abundantly in epidermis (Figure 3H).

Together, these data support a model whereby SOX6 represses transcription in a subset of genes that are essential for epidermal differentiation (Figure 3I).

### Example 22. Results: SOX6 Interacts with SMARCA Chromatin Remodelling Complex and Stalls Epidermal Cell Differentiation

Repression of keratinocyte differentiation by SOX6 is likely to require additional interacting proteins; SOX6 itself lacks a regulatory domain and is known to interact with other proteins in order to execute its functions [20].

To gain insights into SOX6 interacting partners, we performed co-immunoprecipitation followed by mass spectrometry (MS). Towards this we generated HEK293T cell lines with doxycycline-inducible expression of SOX6 fused to an N-terminal MYC tag. The nuclear lysate of doxycycline-treated and non-treated cells were subjected to immunoprecipitation using rabbit IgG, anti-SOX6 and anti-MYC antibodies (Figure 9A). The immunoprecipitated proteins were subsequently subjected to MS analysis to identify the potential interacting partners of SOX6.

After filtering non-specific interactors a total of 26 putative SOX6-interacting proteins that are involved in transcriptional regulation and chromatin remodelling were identified (Figure 9B). Several subunits of human *switch*/*sucrose non-fermentable* (SWI/SNF)-type ATP dependent chromatin remodelling complex were identified in the list of SOX6-interacting proteins. These included SMARCA4 (also known as BRG1), SMARCD2 (also known as BRG1-associated factor, BAF60b), SMARCC1 (BRG1-associated factor, BAF155) and ACTL6a (BRG1-associated factor, BAF53a) [21] (Figure 4A). HELLS (SMARCA6) was also identified as a candidate SOX6 interacting partner.

The SWI/SNF complexes control the accessibility of DNA to sequence-specific transcription factors by nucleosome remodelling, leading to the activation or repression of its target genes [22, 23]. SWI/SNF proteins lack sequence specificity and are known to interact with transcription factors to reach specific target sites.

Therefore, we postulated that interaction of SOX6 with SMARCA proteins may facilitate recruitment of chromatin remodelling complexes to specific genomic loci.

Validation by co-immunoprecipitation/immune-co-localization confirmed the specific interaction of SOX6 with SMARCA4, SMARCA6 and SMARCC1 in keratinocytes (Figure 4B). No co-localization was observed between SOX6 and the negative control protein pinin 1 (PNN1). (Figure 4B). The extent of co-localization was quantified for all tested proteins by calculating the Pearson co-localization co-efficient in Z-stack confocal images (Figure 4C). Loss-of-function studies support the assumption that the transcription factor SOX6 interacts with SMARCC1 and SMARCA4-containing complexes to exert its function during epidermal differentiation. Knockdown of SMARCC1 significantly increases the expression of epidermal differentiation factors *IVL, SPRR2F* and *TGM1* (Figure 4D).

Combining the above findings presents a complete picture of the relationship between miR-335, SOX6, and epidermal differentiation-related proteins in skin maturation.

In healthy skin, proliferating cells in the basal layer of the epidermis express low levels of miR-335. SOX6 is expressed and recruits SMARCA complex components to suppress production of epidermal differentiation factors in the basal epidermis. High levels of miR-335 in suprabasal layers directly block SOX6 translation. This relieves transcriptional repression on *IVL, SPRR2F* and *TGM1,* allowing terminal keratinocyte differentiation to occur.

In contrast, epidermal miR-335 expression is lost in all layers of AD lesional skin. This absence of miR-335 causes sustained expression of SOX6 and the aberrant loss of differentiation-related proteins. The epidermis fails to mature adequately, causing the barrier defect and its associated contribution to allergen sensitization.

### Example 23. Results: Epigenetic Regulation of miR-335 by Histone Deacetylase 2 (HDAC2)

Thus far, we have demonstrated how downregulation of miR-335 leads to a barrier defect coupled with an increased inflammatory microenvironment in AD. However, the molecular mechanisms governing miR-335 expression remain poorly understood.

miR-335 is an intronic miRNA encoded from the second intron of mesoderm-specific transcript (MEST) (Figure 5A). Intronic miRNAs can share the same promoters with their host genes or have their own separate promoters.

Analysis of RNA seq data indicates that miR-335 is expressed in normal human epidermal keratinocytes cells. However, CAGE tags are only present in the upstream region of MEST, but not miR-335, suggesting that miR-335 does not have an independent promoter (Figure 10).

Given the role of miR-335 in directing keratinocyte differentiation and its specific expression in suprabasal layers of epidermis, we investigated the expression of miR-335 in specific conditions which promote keratinocyte differentiation. Histone deacetylase (HDAC) inhibitors have been shown to induce keratinocyte differentiation and recent reports support their role in alleviating AD-like phenotypes in mouse models [24, 25].

We observed that treating N/TERT-1 keratinocytes with sodium butyrate (NaB), a broad-spectrum HDAC inhibitor, caused significant miR-335 upregulation (Figure 5B).

NaB treatment also significantly increased MEST expression, supporting our hypothesis that miR-335 is transcribed in tandem with its host gene (Figure 5C). A significant increase in the enrichment of H3K4ac at the upstream region of MEST and miR-335 upon treatment with NaB further substantiates the role of HDAC as a transcriptional regulator of both miR-335 and MEST (Figure 5D).

However, it is likely that only miR-335 mediates NaB-induced keratinocyte differentiation - knocking down MEST did not significantly affect production of *KRT1 (a* suprabasally expressed marker of keratinocyte differentiation), *IVL,* or *TGM1* (Figure 5E). On the other hand treatment with NaB leads to a significant upregulation of differentiation makers such as IVL, SPRR2F and TGM1 (Figure 5F).

These observations suggest that NaB-induced HDAC inhibition enables miR-335 to be expressed and exert its pro-differentiation effects. By inference, miR-335 suppression may result from HDAC activity, and the use of HDAC inhibitors to restore miR-335 expression in atopic dermatitis could represent a potential therapeutic strategy for restoring the skin barrier defect.

### Example 34. Results: Belinostat Restores Barrier Function and Epidermal Homeostasis via miR-335 Network

HDAC inhibitors (HDACi) can reprogram the cellular machinery to induce cell cycle arrest, differentiation and apoptosis, by altering the acetylation status of an array of substrates, including histones, transcription factors or chaperone proteins. Interestingly, HDAC inhibitors can alter the expression profiles of miRNAs [26].

As expression of miR-335 is regulated by HDACs, we sought to identify HDAC inhibitors which can induce the expression of miR-335.

We treated N/TERT-1 cells with a panel of 42 HDAC inhibitors and identified five HDAC inhibitors which can stimulate the expression of miR-335 and IVL, leading to keratinocyte differentiation. Notably, the HDAC inhibitors that induced miR-335 also displayed growth arrest and spontaneous differentiation, although some of these inhibitors exhibited high toxicity.

We identified belinostat as the potential candidate to target miR-335 based on its significance, consistency and low toxicity (Figure 6A).

Induced expression of miR-335 in N/TERT-1 cells upon treatment with belinostat, was confirmed by *in situ* hybridization (Figure 6B, upper panel). Immuno-cytochemistry on N/TERT-1 cells displayed significant expression of IVL upon treatment with belinostat compared to the control cells (Figure 6B, lower panel). In addition, cornified envelop assay further showed increased number of CEs in cells treated with belinostat compared to control indicating that belinostat can restore epidermal homeostasis by stimulating miR-335 expression (Figure 6C and Figure 6D). These findings emphasize the importance of post-transcriptional regulation by HDAC inhibitors by inducing the expression of specific miRNA.

To test the therapeutic potential of belinostat, we adopted an *ex vivo* human organ culture model, which is more appropriate to assess the effect of individual small molecule drugs through topical penetration or by percutaneous absorption. *Ex-vivo* organ cultures from human elective surgery, skin sections which have normal skin barrier function and a mature stratum corneum were selected for the experiment.

Punch biopsies from these skin samples were cultured at air-liquid interface [27]. Biopsies were topically treated with acetone alone or HDAC inhibitors dissolved in acetone. Quantitative RT-PCR analysis revealed significant expression of miR-335 upon treatment with belinostat compared to other HDAC inhibitors (Figure 6E).

This was further confirmed by *in situ* hybridization wherein belinostat treated skin showed higher expression of miR-335 in epidermal layers compared to control mock treated skin (Figure 6F).

Moreover, immunohistochemistry revealed that treatment with belinostat resulted in a significant increase in the levels of IVL, a bona fide downstream target gene of miR-335 (Figure 6G).

Further, belinostat also induced significant levels of filaggrin (FLG) protein (Figure 11). FLG is a natural moisturising factor, often mutated or downregulated in AD.

All the above suggests that, belinostat can effectively stimulate the expression of miR-335 and potentially restores epidermal homeostasis in a dry skin model, which simulates an AD-like phenotype.

In conclusion, the HDAC inhibitor, belinostat may alleviate inflammation and barrier defect via miR-335 network in AD.

### Example 25. Discussion

Atopic dermatitis is thought to be caused by both genetics and environmental factors. However, the exact molecular mechanisms regulating AD pathophysiology remain elusive.

The two major pathophysiological hallmarks of AD are epidermal barrier dysfunction with altered keratinocyte proliferation/differentiation [28] and infiltration of immune cells in lesions [11, 29, 30]. AD was traditionally thought to an immune-driven disease, whereby altered immune response was proposed as the primary defect, and the defective skin barrier regarded as a secondary effect of local inflammation (the "inside-out hypothesis") [11].

However, increasing evidence supports the opposite "outside-in" hypothesis whereby inherited abnormalities in epidermal structural and enzymatic proteins that impair skin barrier function lead to increased antigen penetration, allergen sensitization, and inflammation [31].

Epidermal differentiation is a multi-step process characterized by tightly controlled and sequential expression of unique sets of genes, which are turned on and off in proliferation- and differentiation-specific manners. These includes genes encoding structural cytoskeletal proteins (KRT5, KRT14, KRT1, KRT10) and proteins required for the development of an effective skin barrier (IVL, TGM1, FLG, LOR, SPRRs).

Skin barrier function is conferred by the stratum corneum, which consists of corneocytes embedded in intercellular multilamellar lipids. Each corneocyte is enclosed by a cornified envelope (CE) and connected to its neighbours by corneo-desmosomes [32]. The CE is formed through crosslinking of insoluble structural proteins, including involucrin (IVL), loricrin (LOR), trichohyalin, envoplakin, periplakin and small proline-rich proteins (SPRRs), through γ-glutamyl ε-lysine bonds formed by transglutaminase (TGM1) [33].

Layers of corneocytes in the stratum corneum provide high resilience, enabling the stratum corneum to perform its function as a mechanical shield against pathogen invasion. This, coupled with the presence of surrounding intercellular lipids, confer water impermeability to the epidermis.

miRNAs are known to play important roles as post-transcriptional gene regulators in skin development and diseases. A growing number of miRNAs, such as miR-203 [34], miR-217 [35], and miR-17 [36] have been implicated in regulating keratinocyte differentiation. Our findings provide insights into the role of miR-335 in the maintenance of epidermal homeostasis.

*In situ* hybridization reveals that miR-335 is abundantly expressed in the differentiated layers of the epidermis. This is consistent with previous reports that miR-335 showed higher expression in terminally differentiated keratinocytes compared to proliferating keratinocytes isolated from human skin [37].

Moreover, our observation that miR-335 enhanced cornified envelope formation by regulating the expression of specific differentiation-associated gene signature all suggests that miR-335 is a critical regulator of keratinocyte differentiation. In AD lesional skin, HDAC-mediated downregulation of miR-335 causes sustained SOX6 expression, which abrogates expression of differentiation-related proteins such as IVL, SPRRs and TGM1. This disrupts differentiation and cornification, creating a barrier defect.

SOX6 has been reported as a multifaceted transcription factor which modulates terminal differentiation of many mesoderm-, ectoderm- and endoderm-derived cell lineages [38]. SOX6, which has long 3'UTR, has been demonstrated to be post-transcriptionally regulated by miRNAs. For example, miR-499 and miR-219 have been shown to regulate SOX6 expression during skeletal muscle and oligodendrocyte differentiation respectively [39, 40].

We did not detect differential expression of miR-499 and miR-219 in our array data on healthy and AD skin samples, which is not unexpected as miR-499 and miR-219 have been established as muscle and brain specific miRNAs respectively.

We found that SOX6 is predominantly expressed in the proliferating layer of the epidermis and SOX6 knockdown enhanced keratinocyte cornification. Our transcriptome analysis revealed that the expression of some keratinocyte differentiation-related genes, such as *CNFN, IVL, SPRR* and *TGM1,* are upregulated upon SOX6 knockdown in keratinocytes, highlighting the role of SOX6 as a transcriptional repressor of keratinocyte differentiation and cornification.

In the absence of specific trans-activation or trans-repressor domain SOX6 interacts with various cofactors, such as components of the transcription machinery and chromatin remodelling proteins to execute its function. For instance, SOX9 and SOX5 have been shown to cooperate with SOX6 in activating three extracellular matrix-related proteins during chondrogenesis: CO12A1 [41], AGC1 [42] and COMP [43]. The transcriptional corepressor CtBP2 has also been shown to be a SOX6 interacting partner in suppressing fibroblast growth factors 3 (*Fgf-3*) transcription [44].

Our results showed that transcriptional repression of specific genes associated with keratinocyte differentiation is mediated by the interaction of SOX6 with chromatin remodelling proteins SMARCA4, SMARCA6 and SMARCC1. Notably, SMARCA4 and SMARCC1 are part of a highly conserved multi-subunit chromatin remodelling complex known as the SWI/SNF family of proteins. The SWI/SNF family of nucleosome-remodelling complexes are known play crucial roles in gene expression. Recent studies have identified transcriptional regulation mediated by biochemically distinct SWI/SNF complexes [45] and possible mechanisms by which SWI/SNF is targeted to specific promoters.

Surprisingly, multiple studies have revealed that, in addition to activation, SWI/SNF is required for transcriptional repression of specific genes including Blimp-1 [46] and cyclin D1 [47]. Here we demonstrate spatio-temporal SOX6-mediated transcriptional repression of specific differentiation associated genes by a distinct SWI/SNF complex.

miR-335 belongs to a cohort of miRNAs regulated by HDACs. Previous studies have demonstrated that multiple HDAC isoforms may regulate miRNA expression [48, 49]. Our ChIP data showed that HDAC1 and HDAC2 were highly enriched in the promoter region of miR-335 and HDAC2 enrichment was reduced when keratinocytes were treated with NaB, suggesting that HDAC2 may regulates miR-335 expression.

We have examined HDAC1 and HDAC2 in our ChIP experiments, whether any other HDACs are involved in miR-335 regulation requires further investigation. Treatment with HDAC inhibitors resulted in an increased expression of keratinocyte differentiation markers, such as *IVL, TGM1* and *SPRR2F.*

Consistent with these observations, other studies showed that treatments with HDAC inhibitors resulted in premature expression of differentiation markers [50, 51]. While the role of HDACs in AD remains to be elucidated, two independent studies have demonstrated that TSA may suppress the development of AD-like symptoms in mice [24, 25].

This prompts us to hypothesize that HDAC inhibitors may be effective in treating AD, partly by upregulating miR-335 expression to induce differentiation-associated transcriptional signature to restore barrier function.

We identified belinostat as a highly consistent HDACi with low toxicity, which can effectively restore miR-335 expression and induce the expression of proteins involved in epidermal differentiation.

We envision that development of a topical formulation for belinostat, may provide a therapeutic approach to ameliorate the barrier defect and alleviate the therapeutically intractable atopic dermatitis.

### REFERENCES

1. Weidinger, S., et al., Atopic dermatitis. Nat Rev Dis Primers, 2018. 4(1): p. 1.
2. Simpson, E.L.M.M., et al., Update on Epidemiology, Diagnosis, and Disease Course of Atopic Dermatitis. Semin Cutan Med Surg, 2016. 35(5 Suppl): p. S84-8.
3. Agrawal, R. and J.A. Woodfolk, Skin barrier defects in atopic dermatitis. Curr Allergy Asthma Rep, 2014. 14(5): p. 433.
4. Trzeciak, M., et al., Expression of Cornified Envelope Proteins in Skin and Its Relationship with Atopic Dermatitis Phenotype. Acta Derm Venereol, 2017. 97(1): p. 36-41.
5. David Boothe, W., J.A. Tarbox, and M.B. Tarbox, Atopic Dermatitis: Pathophysiology. Adv Exp Med Biol, 2017. 1027: p. 21-37.
6. Huang, E. and P.Y. Ong, Severe Atopic Dermatitis in Children. Curr Allergy Asthma Rep, 2018. 18(6): p. 35.
7. Ahn, C. and W. Huang, Clinical Presentation of Atopic Dermatitis. Adv Exp Med Biol, 2017. 1027: p. 39-46.
8. Paller, A.S., et al., The atopic march and atopic multimorbidity: Many trajectories, many pathways. J Allergy Clin Immunol, 2019. 143(1): p. 46-55.
9. Spergel, J.M. and A.S. Paller, Atopic dermatitis and the atopic march. J Allergy Clin Immunol, 2003. 112(6 Suppl): p. S118-27.
10. Hogan, M.B., K. Peele, and N.W. Wilson, Skin barrier function and its importance at the start of the atopic march. J Allergy (Cairo), 2012. 2012: p. 901940.
11. Boguniewicz, M. and D.Y. Leung, Recent insights into atopic dermatitis and implications for management of infectious complications. J Allergy Clin Immunol, 2010. 125(1): p. 4-13; quiz 14-5.
12. Palmer, C.N., et al., Common loss-of-function variants of the epidermal barrier protein filaggrin are a major predisposing factor for atopic dermatitis. Nat Genet, 2006. 38(4): p. 441-6.
13. Weidinger, S. and N. Novak, Atopic dermatitis. Lancet, 2016. 387(10023): p. 1109-1122.
14. Yi, R., et al., Morphogenesis in skin is governed by discrete sets of differentially expressed microRNAs. Nat Genet, 2006. 38(3): p. 356-62.
15. Andl, T., et al., The miRNA -processing enzyme dicer is essential for the morphogenesis and maintenance of hair follicles. Curr Biol, 2006. 16(10): p. 1041-9.
16. Yi, R., et al., DGCR8-dependent microRNA biogenesis is essential for skin development. Proc Natl Acad Sci U S A, 2009. 106(2): p. 498-502.
17. Dickson, M.A., et al., Human keratinocytes that express hTERT and also bypass a p16(INK4a)-enforced mechanism that limits life span become immortal yet retain normal growth and differentiation characteristics. Mol Cell Biol, 2000. 20(4): p. 1436-47.
18. Steven, A. C. and P.M. Steinert, Protein composition of cornified cell envelopes of epidermal keratinocytes. J Cell Sci, 1994. 107 ( Pt 2): p. 693-700.
19. An, C.I., Y. Dong, and N. Hagiwara, Genome-wide mapping of Sox6 binding sites in skeletal muscle reveals both direct and indirect regulation of muscle terminal differentiation by Sox6. BMC Dev Biol, 2011. 11: p. 59.
20. Kamachi, Y., M. Uchikawa, and H. Kondoh, Pairing SOX off: with partners in the regulation of embryonic development. Trends Genet, 2000. 16(4): p. 182-7.
21. Sarnowska, E., et al., The Role of SWIISNF Chromatin Remodeling Complexes in Hormone Crosstalk. Trends Plant Sci, 2016. 21(7): p. 594-608.
22. Kadoch, C. and G.R. Crabtree, Mammalian SWI/SNF chromatin remodeling complexes and cancer: Mechanistic insights gained from human genomics. Sci Adv, 2015. 1(5): p. e1500447.
23. Martens, J.A. and F. Winston, Recent advances in understanding chromatin remodeling by Swi/Snf complexes. Curr Opin Genet Dev, 2003. 13(2): p. 136-42.
24. Kim, T.H., et al., The histone deacetylase inhibitor, trichostatin A, inhibits the development of 2, 4-dinitrofluorobenzene-induced dermatitis in NC/Nga mice. Int Immunopharmacol, 2010. 10(10): p. 1310-5.
25. Shi, Y.L., et al., Histone deacetylases inhibitor Trichostatin A ameliorates DNFB-induced allergic contact dermatitis and reduces epidermal Langerhans cells in mice. J Dermatol Sci, 2012. 68(2): p. 99-107.
26. Scott, G.K., et al., Rapid alteration of microRNA levels by histone deacetylase inhibition. Cancer Res, 2006. 66(3): p. 1277-81.
27. Moll, I., et al., Characterization of epidermal wound healing in a human skin organ culture model: acceleration by transplanted keratinocytes. J Invest Dermatol, 1998. 111(2): p. 251-8.
28. Guttman-Yassky, E., et al., Broad defects in epidermal cornification in atopic dermatitis identified through genomic analysis. J Allergy Clin Immunol, 2009. 124(6): p. 1235-1244 e58.
29. Boguniewicz, M. and D.Y. Leung, Atopic dermatitis: a disease of altered skin barrier and immune dysregulation. Immunol Rev, 2011. 242(1): p. 233-46.
30. Leung, D.Y., New insights into atopic dermatitis: role of skin barrier and immune dysregulation. Allergol Int, 2013. 62(2): p. 151-61.
31. Cork, M.J., et al., Epidermal barrier dysfunction in atopic dermatitis. J Invest Dermatol, 2009. 129(8): p. 1892-908.
32. Candi, E., R. Schmidt, and G. Melino, The cornified envelope: a model of cell death in the skin. Nat Rev Mol Cell Biol, 2005. 6(4): p. 328-40.
33. Nemes, Z. and P.M. Steinert, Bricks and mortar of the epidermal barrier. Exp Mol Med, 1999. 31(1): p. 5-19.
34. Lena, A.M., et al., miR-203 represses 'stemness' by repressing DeltaNp63. Cell Death Differ, 2008. 15(7): p. 1187-95.
35. Zhu, H., et al., MiR-217 is down-regulated in psoriasis and promotes keratinocyte differentiation via targeting GRHL2. Biochem Biophys Res Commun, 2016. 471(1): p. 169-76.
36. Wu, N., et al., The miR-17family links p63 protein to MAPK signaling to promote the onset of human keratinocyte differentiation. PLoS One, 2012. 7(9): p. e45761.
37. Hildebrand, J., et al., A comprehensive analysis of microRNA expression during human keratinocyte differentiation in vitro and in vivo. J Invest Dermatol, 2011. 131(1): p. 20-9.
38. Hagiwara, N., Sox6, jack of all trades: a versatile regulatory protein in vertebrate development. Dev Dyn, 2011. 240(6): p. 1311-21.
39. Nachtigall, P.G., et al., MicroRNA-499 expression distinctively correlates to target genes sox6 and rod1 profiles to resolve the skeletal muscle phenotype in Nile tilapia. PLoS One, 2015. 10(3): p. e0119804.
40. Dugas, J.C., et al., Dicer1 and miR-219 Are required for normal oligodendrocyte differentiation and myelination. Neuron, 2010. 65(5): p. 597-611.
41. Lefebvre, V., P. Li, and B. de Crombrugghe, A new long form of Sox5 (L-Sox5), Sox6 and Sox9 are coexpressed in chondrogenesis and cooperatively activate the type II collagen gene. EMBO J, 1998. 17(19): p. 5718-33.
42. Han, Y. and V. Lefebvre, L-Sox5 and Sox6 drive expression of the aggrecan gene in cartilage by securing binding of Sox9 to a far-upstream enhancer. Mol Cell Biol, 2008. 28(16): p. 4999-5013.
43. Liu, C.J., et al., Transcriptional activation of cartilage oligomeric matrix protein by Sox9, Sox5, and Sox6 transcription factors and CBP/p300 coactivators. Front Biosci, 2007. 12:p. 3899-910.
44. Murakami, A., et al., SOX6 binds CtBP2 to repress transcription from the Fgf-3 promoter. Nucleic Acids Res, 2001. 29(16): p. 3347-55.
45. Raab, J.R., S. Resnick, and T. Magnuson, Genome-Wide Transcriptional Regulation Mediated by Biochemically Distinct SWI/SNF Complexes. PLoS Genet, 2015. 11(12): p. e1005748.
46. Choi, J., et al., The SWI/SNF chromatin remodeling complex regulates germinal center formation by repressing Blimp-1 expression. Proc Natl Acad Sci U S A, 2015. 112(7): p. E718-27.
47. Rao, M., et al., Inhibition of cyclin D1 gene transcription by Brg-1. Cell Cycle, 2008. 7(5): p. 647-55.
48. Koumangoye, R.B., et al., SOX4 interacts with EZH2 and HDAC3 to suppress microRNA-31 in invasive esophageal cancer cells. Mol Cancer, 2015. 14: p. 24.
49. Hsieh, T.H., et al., HDAC inhibitors target HDAC5, upregulate microRNA-125a-5p, and induce apoptosis in breast cancer cells. Mol Ther, 2015. 23(4): p. 656-66.
50. Markova, N.G., et al., Inhibition of histone deacetylation promotes abnormal epidermal differentiation and specifically suppresses the expression of the late differentiation marker profilaggrin. J Invest Dermatol, 2007. 127(5): p. 1126-39.
51. Leon Carrion, S., C.H. Sutter, and T.R. Sutter, Combined treatment with sodium butyrate and PD153035 enhances keratinocyte differentiation. Exp Dermatol, 2014. 23(3): p. 211-4.
52. Sundaram, G.M., et al., 'See-saw' expression of microRNA-198 and FSTL1 from a single transcript in wound healing. Nature, 2013. 495(7439): p. 103-6.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

## Claims

1. An agent capable of up-regulating the expression or activity of miR-335 such as an miR-335 agonist, for use in a method of treatment, prophylaxis or alleviation of atopic dermatitis, wherein the agent comprises belinostat (PubChem CID: 6918638).

2. The agent for use of claim 1, wherein the agent is formulated as a pharmaceutical composition comprising the agent according to claim 1 together with a pharmaceutically acceptable excipient, carrier or diluent.

3. The agent for use according to claim 2, which is formulated to be applied topically.

4. Belinostat (PubChem CID: 6918638) for use in the treatment, prophylaxis or alleviation of atopic dermatitis.

5. An *in vitro* or *ex vivo* method of up-regulating the expression of miR-335 in a cell, the method comprising exposing the cell to belinostat (PubChem CID: 6918638).

## Patentansprüche

1. Mittel, welches imstande ist, die Expression oder Aktivität von miR-335 hochzuregulieren, wie ein miR-335-Agonist, zur Verwendung in einem Verfahren zur Behandlung, Prophylaxe oder Linderung von atopischer Dermatitis, wobei das Mittel Belinostat (PubChem CID: 6918638) umfasst.

2. Mittel zur Verwendung nach Anspruch 1, wobei das Mittel als eine pharmazeutische Zusammensetzung formuliert ist, welche das Mittel nach Anspruch 1 zusammen mit einem pharmazeutisch akzeptablen Hilfsstoff, Träger oder Verdünnungsmittel umfasst.

3. Mittel zur Verwendung nach Anspruch 2, welches dazu formuliert ist, topisch angewendet zu werden.

4. Belinostat (PubChem CID: 6918638) zur Verwendung bei der Behandlung, Prophylaxe oder Linderung von atopischer Dermatitis.

5. *In vitro* oder *ex vivo* Verfahren zum Hochregulieren der Expression von miR-335 in einer Zelle, wobei das Verfahren umfasst, die Zelle Belinostat (PubChem CID: 6918638) auszusetzen.

## Revendications

1. Agent capable de réguler à la hausse l'expression ou l'activité de miR-335 tel qu'un agoniste de miR-335, pour utilisation dans un procédé de traitement, de prophylaxie ou de soulagement de la dermatite atopique, dans lequel l'agent comprend du belinostat (PubChem CID : 6918638).

2. Agent pour utilisation selon la revendication 1, dans lequel l'agent est formulé sous la forme d'une composition pharmaceutique comprenant l'agent selon la revendication 1 avec un excipient, un support ou un diluant pharmaceutiquement acceptable.

3. Agent pour utilisation selon la revendication 2, qui est formulé pour être appliqué par voie topique.

4. Belinostat (PubChem CID : 6918638) pour utilisation dans le traitement, la prophylaxie ou le soulagement de la dermatite atopique.

5. Procédé *in vitro* ou ex *vivo* de régulation positive de l'expression de miR-335 dans une cellule, le procédé comprenant l'exposition de la cellule à du belinostat (PubChem CID : 6918638).
